Europäisches Patentamt

European Patent Office

Office européen des brevets

(11) Publication number: **0 137 869 B1**

# EUROPEAN PATENT SPECIFICATION

(45) Date of publication of patent specification: **14.04.93**

(51) Int. Cl.⁵: **C12N 15/00**, C12P 21/02, C12N 1/00, A23L 3/34, A23K 1/17

(21) Application number: **83110479.9**

(22) Date of filing: **20.10.83**

(54) **Derived nisin producing microorganisms, method of production and use and products obtained thereby.**

(30) Priority: **06.09.83 US 529614**

(43) Date of publication of application: **24.04.85 Bulletin 85/17**

(45) Publication of the grant of the patent: **14.04.93 Bulletin 93/15**

(84) Designated Contracting States: **AT BE CH DE FR GB IT LI LU NL SE**

(56) References cited:
DE-B- 1 180 612
GB-A- 713 251

THE MERCK INDEX, 10TH EDITION, 1983, PAGE 942, REF.NO.6410: "NISIN", MERCK & CO., Rahway, US

JOURNAL OF GENERAL MICROBIOLOGY, vol.88, 1975, pages 189-192, GB P.G. FUCHS et al.: "Possible plasmid nature of the determinant for production of the the antibiotic nisin in some strains of Streptococcus lactis."

(73) Proprietor: **MICROLIFE TECHNICS, INC.**
**1833 57th Street**
**Sarasota Florida 33578(US)**

(72) Inventor: **Gonzalez, Carlos F.**
**4134 Palau Drive**
**Sarasota Florida 33583(US)**
Inventor: **Gryczka, Alfred J.**
**3380 Pin Oaks Street**
**Sarasota Florida 33580(US)**

(74) Representative: **Patentanwälte Grünecker,**
**Kinkeldey, Stockmair & Partner**
**Maximilianstrasse 58**
**W-8000 München 22 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.5x/3.0.1)

CHEMICAL ABSTRACTS, vol.89, no.15, October 9, 1978, page 303, ref.no. 125992u; Columbus, Ohio, US D.J. LeBLANC et al.: " Conjugal transfer of plasmid DNA among oral streptococci."

CHEMICAL ABSTRACTS, vol.89, no.17, October 23, 1978, page 295, ref.no.143101g; Columbus , Ohio, US D.J. LeBLANC et al.: "Transformation of group F streptococci by plasmid DNA." & J. Gen. Microbiol., 1978, 106 (1), 49 - 54

APPLIED AND ENVIROMENTAL MICROBIOLOGY, March 1985, vol.49, no.3,American Society for Microbiology, US, pages 627-633 C.F. GONZALEZ et al.: "Transfer of sucrose-fermenting ability and nisin production phenotype among lactic streptococci."

Natl. Assd. Sci. USA, 1978, 75(7), 3484-3487

Journal of General Microbioligy, vol 88, 1975, 189-192,

Int. Diary Congr. , Proc. 17th. ,Munich ,1966, 633-640

## Description

The present invention relates to novel derived microorganisms and to a method for producing the derived microorganisms which contain foreign DNA encoding for nisin production. In particular, the present invention relates to derived bacteria containing foreign DNA encoding for nisin production.

### Nisin

The Merck Index (8th Edition) at page 6375 generally characterizes nisin as a polypeptide antibiotic produced by *Streptococcus lactis*, citing various publications including Mattick, Hirsch, Nature 154, 551 (1944); Lancet 250, 417 (1946); and 253, 5(1947); Berridge et al., Biochem. J. 52, 529 (1952); and U.S. Pat. 2,935,503(1960). The chemical structure is indicated to contain 34 amino acid residues, eight of which are rarely found in nature, including lanthionine (two alanines bonded to sulfur at the beta-carbons) and beta-methyllanthionine as described by Gross, J. Am. Chem. Soc. 93, 4634 (1971). Nisin is indicated to form crystals from ethanol and to be soluble in dilute acids. It is stable to boiling in acid solution. The Merck Index indicates that nisin is used in food processing and as a preservative, especially for cheese and canned fruits and vegetables.

A chemical structure of one form of nisin has been described by Gross and Morrell of the National Institute of Health in Chem. and Eng. News Page 18 (Sept. 24, 1973). The structure includes alpha-beta unsaturation in amino acids near the terminal amino and acid groups. It is speculated that the activity of nisin is related to reaction of the unsaturated amino acids with the sulfhydryl groups of enzymes in the affected microorganisms. Nisin generally has a published molecular weight range between about 6800 and 7500. Nisin is classed as an antibiotic produced by N-group lactic acid producing *Streptococci*. The inventors prefer the phrase "inhibitory substance" to "antibiotic" where nisin is generated *in situ* in a food by the microorganism, particularly by bacteria.

### Use in Foods

Nisin in foods inhibits clostridial spoilage which is a major problem resulting from food storage. In addition, nisin inhibits psychotrophic bacteria which are particularly a problem with refrigerated foods. Thus nisin inhibits *Streptococci* of Groups A, B, E, F, H, K, M and N, *Staphylococci, Micrococcus, Bacillus* - (some species) *Clostridium, Mycobacterium, Lactobacillus, Octinomyces* and *Erysipelothrix*. Nisin is particularly effective where the food has been partially heat treated. It is not affected by the presence of foods containing blood serum or milk. It is thus useful in settings where these substances are present in substantial amounts. At this time nisin can not be added to foods in the United States but is used in many countries elsewhere in the world; however, approval is being sought in the United States. It should be noted that nisin occurs naturally in fermented food products where nisin producing strains of *Streptococcus lactis* are present, particularly in milk products. Use in foods is described by Reddy et al J. of Food Science 35, 787-791 (1970) and in Reddy et al J. Food Science 40, 314-318 (1975). GB-A-713,251 discloses the use of nisin or nisin-producing bacteria cultures in a process of manufacturing or preserving cheese. Lipinska et al., Int. Diary Congr., Proc. 17th, Munich 1966, 4, 633-640, teache the use of nisin in a mixture containing Streptococcus cremoris, Streptococcus diacetilactis and two strains of Streptococcus lactis for incubation of milk in order to produce cheese. An assay procedure in foods is described by Trainer, J., et al J. Sci Fd Agriculture 15 522-528 (1964). The Assay of Nisin In Foods. Fowler, G. G.; Garvis, B.; Tramer, J., Aplin & Barret Ltd., Yeovil, Somerset, U.K. Technical Series. Society for Applied Bacteriology, 1975, No. 8 pp 91-105.

### Strains

Various strains of *Streptococcus lactis* are known to produce nisin, but *S. cremoris* and *S. lactis* subspecies *diacetilactis* do not. Such strains are described in: McClintok, H. et al. J. Dairy Research 19, 187-193 (1952); Campbell, L. L. et al Food Tech 13:462-464 (1959); Hurst J. Gen Microbiology 44:209-220 (1966); Mattick and Hirsch 12th Intern. Dairy Congress 2 (Sect 3) 546-550 (1949); Campbell et al, Food Preservation by Use of Chemicals 110-119 (about 1960); McClintock et al, J. Dairy Res 19:187-193 (1952) (French); Rayman, Applied and Environmental Tech 41:375, 380 (1981); Scott et al Journal of Food Science 40:115-126 (1981); and in U.S. Patent Nos. 2,935,503; 3,093,551; 2,785,108; and 3,295,989.

Various technical articles have appeared describing nisin producing *Streptococcus lactis* strains. Includes are Geis A, et al Applied Environmental Microbiology 45:205-211 (1983); Kozak et al J. of Gen.

3

Microbiology 83:295-302 (1974); Pack, et al, J. Bacteriology 149 420-425 (1982); McKay, L. et al Applied and Environmental Microbiology 40:84-91 (1980); Hurst A, J. Gen Microbiology 44:209-220 (1966); Scherwitz, K., et al., Applied and Environmental Microbiology 45:1506-1512 (1983); LeBlanc, D., et al., J. of Bacteriology 137:878-884 (1979) and LeBlanc D., et al in Plasmids and Transposons.

Fuchs et al Journal of General Microbiology 88: 189-192 (1975) describe some observations indicating that the nisin coding gene may be located on a streptococcal plasmid.

Environmental Effects and Maintenance Mechanisms, Edited by Colin Stuttard and Kenneth R. Rozee, Academic Press, 31-41 (1980). Some of these publications describe the plasmids in *Streptococcus lactis* and in particular a 28 Mdal plasmid which may encode for nisin production. Le Blanc et al Proc. Natl. Acad. Sci. USA 75: 3484-3487 (1978) disclose "conjugal" transfer of plasmid DNA among oral Streptococci; however, none of the publications describe the transfer of the nisin encoding plasmid to non-nisin producing recipient bacteria or to other microorganisms.

A summary regarding nisin appears in Hurst, A., Advances Applied Microbiology 27:85-123 (1981). This publication describes what is generally known about nisin.

A major problem described in the prior art is the phage susceptibility of nisin producing strains of *Streptococcus lactis*. Another problem is that the nisin is expressed at relatively low levels in the naturally occurring strains of *Streptococcus lactis* and the strains are difficult to grow. Still another problem is that nisin alone has some limitations of inhibitory activity against food spoilage microorganisms.

It is an object of the present invention to provide nisin producing derived microorganisms containing foreign DNA which encodes for nisin production. It is further an object of the present invention to provide a large collection of nisin producing derived microorganisms which have unrelated phage susceptibilities and which generate relatively large amounts of nisin. Also, provided are nisin producing derived microorganisms which can be adapted to become phage insensitive derivatives by direct challenge with phage for selection of insensitive clones without loss of desired functional characteristics, particularly nisin production. Further still it is an object of the present invention to provide nisin producing derived microorganisms which can be combined with other inhibitory substance producing microorganisms.

The present invention broadly relates to a novel nisin producing derived microorganism which contains foreign DNA, either chromosomal or extrachromosomal, which encodes for nisin production, which foreign DNA was obtained from a nisin producing donor Streptococcus and which derived microorganism is obtainable by mating a nisin-producing donor bacterium with a nisin-sensitive recipient bacterium resulting in the nisin-producing derived microorganism. The present invention particularly relates to a novel nisin producing derived bacterium.

The following definitions are used herein:

(1) the phrase "donor microorganism" means a parental Streptococcus strain containing transferable DNA which encodes for nisin production, preferably strains of *Streptococcus lactis* bacteria which naturally produce nisin.

(2) The phrase "recipient microorganism" means a parental bacterium strain which does not naturally produce nisin or which produces relatively low amounts of nisin and may or may not be sensitive to (inhibited by) nisin.

(3) The phrase "derived microorganisms" means nisin producing strains that result from introducing foreign DNA into a recipient bacterium, preferably from mating a donor bacterium which is nisin producing with a recipient bacterium which is non-nisin producing or low nisin producing to increase nisin production.

(4) The phrase "foreign DNA" means DNA which does not naturally occur in the recipient bacterium. The foreign DNA is introduced into the recipient bacterium by the method of the present invention.

(5) The phrase "inhibitory substance" means an antimicrobial agent including nisin produced by a microorganism which prevents the growth of other microorganisms.

The present invention particularly relates to a nisin producing derived bacterium selected from *Streptococcus* species, *Pediococcus* species, *Lactobacillus* species, *Propionibacterium* species, *Micrococcus* species, *Leuconostoc* species, *Staphylococcus* species, *Clostridium* species *Flavobacterium* species, *Brevibacterium* species, *E. coli* and *Pseudomonas* species.

Conventional conjugal mating of donor and recipient bacteria can be used for the transfer of the foreign DNA. The present invention also relates to a method for engineering a nisin producing derived microorganism which comprises:

(a) transferring DNA which DNA encodes for nisin production in a donor Streptococcus to a recipient bacterium which is sensitive to nisin by mating a donor bacterium with a recipient bacterium to result in a nisin producing derived microorganism, wherein the DNA is foreign to the recipient bacterium and encodes for nisin production in the recipient bacterium; and

4

(b) isolating the nisin producing derived microorganism.

The present invention also relates to a nisin containing product elaborated by the nisin producing microorganism with the foreign DNA. The nisin containing product can contain in addition other inhibitory substances preferably those simultaneously elaborated by the nisin producing derived microorganism.

The present invention further relates to two or more of the nisin producing derived microorganism strains containing the foreign DNA as a mixture, which strains have different phage susceptibilities so as to enable the production of nisin without stringent anti-bacteriophage precautions. The strains can be packaged mixed or separately.

Finally the present invention relates to the use of nisin producing microorganisms in the manufacture of a preparation, containing the microorganisms alone or in a mixture with other microorganisms, for use in the treatment of animals.

The present invention is based upon the fact that DNA encoding for nisin production in a donor microorganism can be incorporated into a recipient microorganism as foreign DNA to provide a new nisin producing derived microorganism. The resulting nisin producing derived microorganism is able to preserve foods and other materials and to provide inhibitory substances in animals, particularly birds and mammals, to maintain or improve their health. The nisin producing derived microorganisms containing the foreign DNA can be combined with non-nisin producing, nisin insensitive bacteria or other microorganisms preferably those which can produce other inhibitory substances structurally different from nisin.

Sometimes nisin can be encoded for in the naturally occurring nisin producing *Streptococcus lactis* by a plasmid. Also the plasmid DNA encoding for nisin can be incorporated into the chromosome of the recipient bacterium by natural ligation processes, which makes the removal of the characteristic in the derived microorganism difficult. Thus, in the present invention, the foreign DNA after transfer can be chromosomal or extrachromosomal in the derived microorganism.

The plasmid can be transferred by conjugal mating of related or unrelated genera and species. The resulting nisin producing derived microrganism may have multiple copies of the foreign DNA in each cell which can greatly increase the amount of nisin produced per cell subject to cellular limitations as to concentration of nisin.

As is known in the art, the derived microorganisms are provided in various preserved forms which increase viability during storage and shipment. Such forms include: liquid non-concentrated or concentrated cultures, frozen or non-frozen cultures and stabilized dried or lyophilized cultures. The cultures usually contain biologically pure forms of the derived microorganisms. Amounts of the microorganism over about $10^6$ cells per gram, and preferably over about $1 \times 10^9$ cells per gram in preserved form are preferred. Generally, concentrations over $10^9$ cells per gram require means for removal of liquid growth medium from the cells usually by centrifugation or reverse osmosis. Various stabilizing agents such as glycerol, milk powder and the like are used for freezing and lyophilization. The derived microorganism cells are usually grown in a growth medium including a source of carbon, nitrogen and essential minerals. All of this is well known to those skilled in the art as can be seen from the large body of patent art.

The present invention particularly relates to a biologically pure nisin and lactic acid producing derived bacterium which is not sensitive to nisin and containing plasmid derived DNA, encoding for nisin production from a donor nisin producing *Streptococcus lactis* wherein the DNA has been transferred to a nisin sensitive recipient bacterium resulting in the nisin and lactic acid producing derived bacterium. The recipient bacterium is preferably *Streptococcus lactis* subspecies *diacetilactis*. The donor bacterium for *S. lactis* is preferably *Streptococcus lactis* ATCC 11,454 which transfers the nisin trait only to isogenic strains as can be seen from Table 1. Sucrose utilization also is transferred with the nisin trait. A related known nisin producing strain is NRRL-B-15,470 (NIRD 1404) which could be used. The derived donors are *S. lactis*, NRRL-B-15,459 and NRRL-B-15,460 which can transfer the nisin, sucrose trait intergenetically. All of the nisin producing derived microorganisms which are transconjugants can potentially be used as donors of the nisin trait. The reason may be that the plasmid encoding for nisin and sucrose may have been modified in the primacy transfer. The intergenetic recipient bacterium is preferably *Streptococcus lactis* subspecies *diacetilactis* NRRL-B-15,005, NRRL-B-15,006, NRRL-B-15,018, NRRL-B-12,070 (DRC3, cit⁻,lac⁺), and NRRL-B-12071 (DRC3, lac⁺, cit⁻), and *S. lactis* NRRL-B-15,454, NRRL-B-15,452, NRRL-B-15,455, NRRL-B-15,456, NRRL-B-15,457. The resultant nisin producing derived bacterium with the foreign DNA is preferably selected from *Streptococcus lactis* subspecies *diacetilactis* NRRL-B-15,464, (and related transconjugants from similar matings 15,465, 15,466 and 15,467), 15,468 and 15,469 and isogenic bacterial derivatives thereof as described more fully hereinafter with the appropriate antibiotic resistance marker(s) removed by selection.

The present invention particularly relates to a biologically pure nisin and lactic acid producing bacterium which is not sensitive to nisin and which is derived by conjugal mating of a nisin producing *Streptococcus*

*lactis* donor bacterium containing a plasmid, which codes for the nisin production in the donor bacterium, with a non-nisin producing and nisin sensitive lactic acid producing recipient bacterium. The conjugal mating results in the nisin and lactic acid producing derived bacterium wherein, in some instances, the original plasmid from the donor encoding for nisin production can not be removed as a plasmid from the nisin and lactic acid producing derived bacterium because of recombination with the host chromosome of the recipient bacterium. The recipient bacterium can be inhibited by nisin prior to the transfer. The plasmid which encodes for nisin production (and sucrose fermentation) from the donor bacterium preferably measures about 29 Mdal in length and may exist as 29 or altered form i.e. reduced or enlarged or recombined with host chromosome DNA in the derived bacterium.

The present invention particularly relates to the method for producing a nisin and lactic acid producing derived bacterium which is not sensitive to nisin and which comprises: conjugally mating a nisin producing *Streptococcus lactis* donor bacterium containing a plasmid which codes for the nisin production in the donor bacterium with a non-nisin producing and nisin sensitive lactic acid producing recipient bacterium, and isolating the nisin and lactic acid producing derived bacterium produced by the mating.

Further, the present invention relates to an improvement in a method of preserving a material such as a food by incorporating a lactic acid producing derived bacterium into the material which comprises incorporating in the material a nisin and lactic acid producing derived bacterium which is not sensitive to nisin and containing DNA derived from a plasmid which codes for nisin production from a nisin producing donor *Streptococcus lactis* and which has been transferred to a nisin sensitive recipient bacterium to produce the nisin and lactic acid producing derived bacterium. The foreign DNA encodes for nisin production and is either integrated into the chromosome or extrachromosomal in the nisin producing derived bacterium. The materials can include all sorts of microbially degradable products for agricultural, industrial, food and other uses including silage, cutting oil and foods. The foods include fresh meat, comminuted, fermented and non-fermented meat; fresh chicken, fish, milk and other dairy products, commercially prepared salads, vegetables, dressings, sauces and other foods subject to spoilage during refrigerated storage.

Also, the present invention particularly relates to an improved preserved material produced by incorporating into the material a lactic acid producing derived bacterium or a product produced by the derived bacterium, which comprises (1) a material containing a nisin and lactic acid producing derived bacterium, which is not sensitive to nisin and containing DNA derived from a plasmid which encodes for nisin production from a nisin producing *Streptococcus lactis* donor bacterium which has been transferred to a nisin sensitive recipient bacterium to produce the nisin and lactic acid derived bacterium or (2) incorporating in the material a nisin containing product of such derived bacterium or (3) incorporating in the material a nisin containing product of such derived bacterium along with a microorganism which produces other inhibitory substances in addition to nisin.

EXAMPLE 1

In order to determine if sucrose utilization and nisin production could be transferred by conjugation, *S. lactis* ATCC-11454 (utilizes sucrose and produces nisin) was used as the donor strain in a mating experiment where *S. lactis* subsp. *diacetilactis* and *S. lactis* (neither strain utilizes sucrose and neither strain produce nisin) were the recipient strains. The mating conditions were as described by Gonzalez and Kunka, (Gonzalez, Carlos F., and Blair S. Kunka. Appl. Environ. Microbiol 46:128-132 (1983)) except that the mating filters were not overlaid with agar. Instead, the filter with the cells facing the agar surface were placed in a BBL™ - Anaerobic Jar with $CO_2/H_2$ atmosphere for 3 to 4 hours. Transconjugants were selected for the chromosomal resistance of the recipient and ability to utilize sucrose or resistance to nisin (1000 units/ml).

An isogenic strain of *S. lactis* ATCC 11,454 was constructed. Strain *S. lactis* ATCC-11,454 (utilizes lactose and sucrose, and produces nisin) was temperature cured of a resident 29 Mdal plasmid by exposure to an elevated growth temperature (40°C). This cured strain utilized lactose but did not utilize sucrose and did not produce nisin. Subsequently, this strain was subjected to an additional heat curing at 40°C to remove an additional resident 32 Mdal plasmid to result in constructed strain *S. lactis* ATCC 11,454, which was lactose negative (Lac⁻), sucrose negative (Suc⁻) and did not produce nisin (Nis⁻). This constructed strain was designated as *S. lactis* NRRL-B-15,453 (Lac⁻, Suc⁻, Nis⁻). NRRL-B-15,453, then was exposed to increasing concentrations of streptomycin to obtain a chromosomal mutation for the antibiotic at a concentration of 1000 g/ml. This NRRL-B-15,453 Sm$^r$ (streptomycin resistant) strain was designated as NRRL-B-15,454. Strain NRRL-B-15,454 was then used as a recipient in the conjugal mating experiments.

Table 1 describes the experiments and the results. In these experiments, transfer of sucrose utilization and nisin production only was observed with the isogenic recipient *S. lactis* NRRL-B-15,454. Transfer of sucrose utilization and nisin production intragenerically from *S. lactis* NRRL-B-15,454 to *S. lactis* NRRL-B-15,452 and *S. lactis* subsp. *diacetilactis* NRRL-B-15,006-Sm$^r$ was not observed.

Table 1

| Intraspecies Conjugal Matings | | |
|---|---|---|
| Donor | Recipients | Transfer frequencies per donor |
| *S. lactis* ATCC 11454 | *S. lactis* NRRL-B-15452 | NO |
| " | *S. lactis* NRRL-B-15454 | $1 \times 10^{-5}$ |
| " | *S. lactis* subsp. *diacetilactis* NRRL-B-15006-Sm$^r$ | NO |

NO - Not Detected Sm$^r$ - Resistance to streptomycin at concentration of 1000 $\mu$g/ml.

Mating conditions as described by Gonzalez and Kunka, 1983 (Appl. Environ. Microbiol. 46:81-89). See Example 1 in text for exception.

Transfer frequency is expressed as the number of nisin resistant and nisin producing colonies per donor colony forming units (CFU). Donor CFU were determined before mating. Transconjugants were selected for the chromosomal resistance of the recipient and ability to utilize sucrose or resistance to nisin (1000 units/ml).

EXAMPLE 2

*S. lactis* ATCC 11,454 was exposed to increasing concentrations of rifamycin to obtain a rifamycin resistant (Rif$^r$) mutant (resistant to 400 $\mu$g/ml). The mutant was used as a recipient in a conjugal mating experiment with a *S. sanguis* V683 strain which contains the conjugative plasmid pIP501. The resultant transconjugant (*S. lactis* ATCC 11,454 Rif$^r$ (pIP501)) was isolated and was designated as *S. lactis* NRRL-B-15,458. *S. lactis* NRRL-B-15,458 along with *S. lactis* ATCC 11,454 were used as donors in isogenic and intrageneric matings to determine conjugal transfer of the nisin genes. The mating conditions for the experiments were as described in Example 1 above.

Table 2 describes results of matings in which strain *S. lactis* NRRL-B-15,458 was used as a donor and *S. lactis* NRRL-B-15,452, *S. lactis* NRRL-B-15,454 (an isogenic strain of *S. lactis* ATCC 11,454) and *S. lactis* subsp. *diacetilactis* NRRL-B-15,006-Sm$^r$ were used as recipients. Transer of plasmid pIP501 was observed to occur in two *S. lactis* strains, while no transfer was detected to *S. lactis* subsp. *diacetilactis*. Transfer of sucrose utilization and nisin production was observed only to isogenic strain *S. lactis* NRRL-B-15,454 and was at a frequency of $1.4 \times 10^{-3}$.

Table 2

| Conjugal Transfer of Nisin Genes | | | |
|---|---|---|---|
| Donor | Recipient | Transfer frequency per donor | |
| *S. lactis* NRRL-B-15458 | *S. lactis* NRRL-B-15452 | pIP501 $2 \times 10^2$ | Nisin NO |
| " | *S. lactis* NRRL-B-15454 (isogenic strain) | $2.7 \times 10^{-5}$ | $1.4 \times 10^{-3}$ |
| " | *S. lactis* subsp. *diacetilactis* NRRL-B-15006-Sm$^r$ | NO | NO |

NO - Not Detected

Mating conditions as described in Example 1

When *S. lactis* NRRL-B-15,458 was mated with *S. lactis* NRRL-B-15,454 (isogenic strain) two different phenotypes of transconjugants were obtained. One phenotype was lactose negative, sucrose positive and produced nisin. This isolate was designated as *S. lactis* NRRL-B-15,459. The other transconjugant was lactose negative, sucrose positive, produced nisin and contained plasmid pIP501. This isolate was designated as *S. lactis* NRRL-B-15,460.

These derived strains, unlike the donor strain, were streptomycin resistant, rifamycin sensitive and did not produce acid from lactose. However, unlike the recipient parental strain the derived strains were now nisin producers and were able to utilize sucrose as a source of carbon to produce acid. Therefore, only isogenic transfer of the sucrose utilizing nisin producing trait was observed by strains *S. lactis* ATCC 11,454 and its pIP501 derivative *S. lactis* NRRL-B-15,458.

EXAMPLE 3

*S. lactis* NRRL-B-15,459 was tested for ability to transfer nisin production to *S. lactis* NRRL-B-15,452. Recipient strain NRRL-B-15,452 was negative for production of nisin, sensitive to nisin and unable to utilize sucrose as a source of carbon to produce acid. The mating conditions were as described in Example 1 and results are described in Table 3.

Table 3

| Intrageneric Transfer of Nisin and Sucrose Utilization Genes | | |
|---|---|---|
| Donor | Recipient | $Nis^+$, $Suc^+$ Transconjugants per donor |
| *S. lactis* NRRL-B-15459 | *S. lactis* NRRL-B-15452 | $6 \times 10^{-5}$ |
| Conditions as described in Table 1<br>$Suc^+$ = Sucrose positive; able to utilize sucrose to produce acid.<br>$Nis^+$ = Nisin positive; able to produce nisin.<br>*S. lactis* NRRL-B-15,459 was able to transfer nisin production, resistance and the ability to utilize sucrose to a non-isogenic strain of *S. lactis* NRRL-B-15,452. | | |

EXAMPLE 4

*S. lactis* NRRL-B-15,460 was tested for ability to transfer nisin production to *S. lactis* NRRL-B-15,452, *S. lactis* subsp. *diacetilactis* NRRL-B-15,455 and NRRL-B-15,456. Recipient strains NRRL-B-15,452, NRRL-B-15,455 and NRRL-B-15,456 are negative for production of nisin, sensitive to nisin and unable to utilize sucrose as a source of carbon to produce acid.

The mating conditions were as described in Example 1 and the results are described in Table 4.

Table 4

Intrageneric Transfer of Nisin Genes and Sucrose Utilization Genes

| Donor | Recipient | | Transfer Frequency per donor | Strain designation of representative transconjugant | Transconjugant Phenotype |
|---|---|---|---|---|---|
| | Strain | Phenotype | | | |
| S. lactis NRRL-B-15460 | S. lactis NRRL-B-15452 | Suc⁻,Nis⁻ | $3.5 \times 10^{-5}$ | NRRL-B-15461 (pIP501 neg.) | Suc⁺,Nis⁺ |
| " | S. lactis NRRL-B-15452 | Suc⁻Nis⁻ | — | NRRL-B-15462 (pIP501 pos.) | Suc⁺,Nis⁺ |
| " | S. lactis subsp. diacetilactis NRRL-B-15455 | Suc⁻,Nis⁻ | $2 \times 10^{-6}$ | NRRL-B-15464 | Suc⁺,Nis⁺ |
| " | S. lactis subsp. diacetilactis NRRL-B-15456 | Suc⁻,Nis⁻ | $2 \times 10^{-7}$ | NRRL-B-15469 | Suc⁺,Nis⁺ |
| " | S. lactis subsp. diacetilactis NRRL-B-15005 -Fusʳ | Suc⁻,Nis⁻ | $1 \times 10^{-6}$ | NRRL-B-15005 Fusʳ,Suc⁺,Nis⁻ | Suc⁺,Nis⁺ |

Conditions and selection as described in Table 1.
Suc⁺ = Sucrose positive; able to utilize sucrose to produce acid.
Nis⁺ = Nisin positive; able to produce nisin.
Nis⁻ = Nisin negative; unable to produce nisin.
Fusʳ = Resistance to fusidic acid at concentration of 20 µg/ml
Lac⁻ = Lactose negative. Not able to utilize lactose to produce acid.
neg. = negative
pos. = positive

S. lactis NRRL-B-15,460 was able to transfer nisin production, nisin resistance and ability to utilize sucrose to a non-isogenic strain of S. lactis NRRL-B-15452 and to S. lactis subsp. diacetilactis NRRL-B-15455.

Many transconjugant, derived strains were isolated from the conjugal matings described in Examples 3 and 4. Derived S. lactis NRRL-B-15461 is typical of a transconjugant of S. lactis NRRL-B-15452. Derived S. lactis subsp. diacetilactis NRRL-B-15469 is typical of a transconjugant of S. lactis subsp. diacetilactis NRRL-B-15456.

Table 5 summarizes phenotypic charactistics of transconjugant strains resulting from matings described in Example 4. Transconjugants obtained from each mating were analyzed for selected and unselected markers. Analysis of plasmid content of transconjugants confirmed them as recipient types. Additionally transconjugants were tested for sensitivity to their respective recipient homospecific phages. The transconjugants were lysed by their homospecific phages while donor controls showed no sensitivity.

Table 5

| Phenotypic characteristics of Parental Recipient Strain and Derived Strains of Table 4 Matings | | | | |
|---|---|---|---|---|
| Strain # | status | Phenotypic Characteristics | | |
| | | Nisin Production | Nisin Resistance | Sucrose Utilization |
| *S. lactis* NRRL-B-15452 | Parental Recipient | - | - | - |
| *S. lactis* NRRL-B-15461 | Derived | + | + | + |
| *S. lactis* subsp. *diacetilactis* NRRL-B-15455 | Parental Recipient | - | - | - |
| *S. lactis* subsp. *diacetilactis* NRRL-B-15464 | Derived | + | + | + |
| *S. lactis* subsp. *diacetilactis* NRRL-B-15456 | Parental Recipient | - | - | - |
| *S. lactis* subsp. *diacetilactis* NRRL-B-15469 | Derived | + | + | + |
| Nisin production - Assay (see text) | | | | |
| Nisin resistance - Growth on medium containing Nisaplin™ at 1000 $\mu$g/ml. | | | | |
| Sucrose - utilization - medium BM containing sucrose at .5% (Gonzalez and Kunka Appl. and Environ. Microbiol. 46: 81-89, 1983). | | | | |

EXAMPLE 5

*S. lactis* subsp. *diacetilactis* NRRL-B-15464, a transconjugant, derived strain from a first mating subsequently was used as a donor in an isogenic mating with *S. lactis* subsp. *diacetilactis* NRRL-B-15018 Sm$^r$ (Streptomycin resistant) and *S. lactis* subsp. *diacetilactis* NRRL-B-15005 Fus$^r$ (fusidic acid resistant).

Table 6 describes the results of the mating experiments. Transfer of sucrose utilization and nisin production traits were observed in the two isogenic recipient strains. Transconjugants from each mating were examined for their plasmid content. Additionally, analyses for non-selected traits were conducted. Transconjugants were confirmed as recipient types.

Table 6

Intraspecies Transfer of Sucrose Utilization and Nisin producing genes

| Donor | Recipient Strain | Recipient Phenotypes | Nisin Transfer Frequency | Strain designation of representative Transconjugant | Transconjugant Phenotype |
|---|---|---|---|---|---|
| S. lactis subsp. diacetilactis NRRL-B-15464 | S. lactis subsp. diacetilactis NRRL-B-15018-Sm$^r$ | Suc$^-$,Nis$^-$ | 1.77x10$^{-5}$ | S. lactis subsp. diacetilactis SLA3.18 | Suc$^+$,Nis$^+$ |
| S. lactis subsp. diacetilactis NRRL-B-15464 | S. lactis subsp. diacetilactis NRRL-B-15005-Fus$^r$ | Suc$^-$,Nis$^-$ | 4.5 x10$^{-7}$ | S. lactis subsp. diacetilactis SLA3.42 | Suc$^+$,Nis$^+$ |

Conditions and selectdion as described in Table 1 Suc$^+$ = Sucrose positive; able to utilize sucrose to produce acid.

Nis$^+$ = Nisin positive; able to produce nisin.

Nis$^-$ = Nisin negative; unable to produce nisin.

Sm$^r$ = Resistance to streptomycin at concentration of 1000 µg/ml.

Fus$^r$ = Resistance to fusidic acid at concentration of 20 µg/ml.

EXAMPLE 6

Production of nisin by strain S. lactis subsp. diacetilactis NRRL-B-15464 in comminuted meat incubated at 10°C.

To determine production of nisin by individual colonies of test strains, the colonies were replicated in petri dishes containing peptonized milk agar which had previously been flooded with an 18 hour culture of S. cremoris ATCC 14365. Strain ATCC 14365 is a nisin sensitive strain used to assay nisin production (J. Gen. Microblol. 4:71, 1950). After 18 hours of incubation, the appearance of zones of inhibition is indicative of nisin production. The method is that of Kozak et al J. Gen. Microbiol. 83:295-302 (1974).

The nisin content of meat was determined by "The Quantitative Agar Diffusion Assay used for the estimation and differentiation of nisin in food" as described by Fowler et at. (The Assay of Nisin in Foods.

Fowler, G.G., Jarvis, B., Tramer, J. Aplin & Barret Ltd., Yeovil, Somerset, UK, Technical Series, Society for Applied Bacteriology, 1975, No. 8, pp. 91-105.

A standard curve was prepared by adding a known concentration of purified nisin to meat. The meat sample was then treated by the method of Tramer et al. The food extract was assayed by the agar diffusion method and a log nisin concentration vs. zone diameter size curve was plotted. *S. cremoris* ATCC 14365 was used as the indicator organism. Additionally, strain *S. lactis* ATCC 11454, a nisin producing and therefore nisin insensitive, also was used as an indicator organism.

Highly purified nisin (obtained from Aplin & Barrett, Ltd.) was added to meat samples and nisin concentrations were calculated using a standard curve.

The inoculum of test organism was prepared by growing NRRL-B-15464 in 500 ml M17 broth (Terzaghi and Sandine Appl. Microbiol. 29:807-813, 1975) for 18 hours. Cells were obtained and washed twice by centrifugation at 10,000 RPM at 10°C for 10 minutes and resuspended in Standard Methods phosphate buffer (E. H. Marth, (Ed.) Standard Methods for Examination of Dairy Products, 14th ed. p. 62, Amer. Public Health Assoc. Washington, D. C. Cell suspension was adjusted spectophotometrically to approximately $1.5 \times 10^9$ colony forming units/ml. Actual number was determined to be $1.7 \times 10^9$ CFU/ml. A fresh picnic (pork) (skin on bone in) was purchased from a local meat supply company. The skin was aseptically removed and the meat comminuted in a sterilized meat chopper. The meat then was apportioned into 50 and 100 g quantities and placed in sterile containers. Containers were set up as follows:

A. Eleven containers with 100 g meat and glucose added to a final concentration of 0.5% by weight. For analysis, these samples were split into two aliquots. One aliquot was assayed without further treatment. The remaining aliquot was used to construct a standard curve by adding a known amount of purified nisin (100-200 units/g of meat).

B. Three containers with 50 g of meat glucose to a final concentration of 0.5% by weight of meat and 100 units of added nisin per gram of meat. This sample was assayed daily for nisin.

C. Eleven containers with 100 g of meat, glucose to a final concentration fo 0.5% by weight of meat and strain *S. lactis* NRRL-B-15464 to a final concentration of $1.7 \times 10^7$ CFU/g of meat. Results are shown on Table 7.

Table 7

Production of Nisin by Derived strain *S. lactis* subsp. *diacetilactis* NRRL-B-15464 Inoculated into Comminuted meat which was incubated at 10°C.

| Day | Nisin Content Express As units per gram of meat | | | Organoleptic Evaluation | | |
|---|---|---|---|---|---|---|
| | Control Meat No Nisin No culture | Meat with Added Nisin | Meat with added NRRL-B-15464 | Control Meat No Nisin No culture | Meat with Added Nisin | Meat with added NRRL-B-15464 |
| 0 | 0 | 133 | 0 | 0 | 0 | 0 |
| 1 | 0 | ND | 0 | ND | 0 | 0 |
| 2 | 0 | 23 | 0 | 0 | 0 | 0 |
| 3 | 0 | 5 | 29 | 1 | 0 | 0 |
| 4 | 0 | ND | 156 | 3 | ND | 0 |
| 5 | 0 | ND | 140 | 3 | ND | 0 |
| 6 | 0 | ND | 115 | 3 | ND | 0 |
| 7 | 0 | ND | 131 | 4 | ND | 0 |
| 8 | 0 | ND | 65 | 4 | ND | 0 |
| 9 | 0 | ND | 72 | 4 | ND | 0 |
| 10 | 0 | ND | 42 | 4 | ND | 2 |

Sample assayed by agar diffusion method as described in text.

ND - not determined

Organoleptic scale =

0 - fresh, clean meat aroma, good red color
1 - slight off aroma, good red color
2 - slight off aroma, fair red color
3 - off aroma, poor red color
4 - putrid aroma, slimy appearance, no red color

EXAMPLE 7

Nisin content in milk was determined by the method used for meat described in Example 6.

The inoculum of test organisms was prepared by growing microorganisms in M17 broth for 18 hours. Cells were obtained and washed twice by centrifugation at 10,000 rpm at 10°C/10 minutes and resuspended in Standard Methods phosphate buffer pH7.2. Suspension was adjusted spectrophotometrically to $1.5 \times 10^8$ colony forming units/ml. Non-fat dry milk (10% w/v) was steamed for 30 minutes cooled and glucose added to a final concentration of 0.5% by weight of milk. Fifty ml of milk-glucose was inoculated at a rate of approximately $10^6$ CFU/ml and incubated at 32°C. Samples were assayed daily.

13

Nisin assay protocol: (1) 30 ml of sample was assayed (2) the pH was adjusted to 2 with 5N HCl (3) The sample was boiled for 5 minutes (4) Chilled (5) The sample was centrifuged 10,000 rpm for 10 minutes at 10°C and (4) Supernatant was assayed by Quantitative Agar Diffusion Method (see above). The assay organism was ATCC 14365 which is sensitive to nisin. ATCC 11454 is insensitive to nisin and a nisin producing strain. A standard curve was constructed on a daily basis by adding nisin to a milk sample at 100 units/ml. The results are shown on Table 8.

Table 8

Production of Nisin by Parental and Derived Strains in Milk Incubated at 32°C

| Strain | Status | Units of Nisin Per ml of Milk | | | |
| --- | --- | --- | --- | --- | --- |
| | | Day 0 | Day 1 | Day 2 | Day 3 |
| S. lactis NRRL-B-15455 | P | 0 | 0 | 0 | 0 |
| S. lactis subsp. diacetilactis NRRL-B-15464 | D | 0 | 39 | 54 | 122 |
| S. lactis ATCC 11454 | P | 0 | 45 | 128 | 109 |
| S. lactis NRRL-B-15452 | P | 0 | 0 | 0 | 0 |
| S. lactis NRRL-B-15461 | D | 0 | 10.5 | 49.3 | 44.8 |
| S. lactis subsp. diacetilactis NRRL-B-15456 | P | 0 | 0 | 0 | 0 |
| S. lactis subsp. diacetilactis NRRL-B-15469 | D | 0 | 30 | 182 | 209 |

* = units/ml
P = parental
D = Derived

Strains NRRL-B-15455, NRRL-B-15452, and NRRL-B-15456 represent parental type strains and strains NRRL-B-15464, NRRL-B-15461 and NRRL-B-15469 represent their derived strains repsectively.

Assay as described in text.

The derived micoorganisms can produce nisin (and possibly other substances which inhibit microorganisms) at refrigeration temperatures which is a distinct advantage in the preservation of foods. This is shown by the results in Table 7 where control meat without a derived microorganism started to spoil at day 3 and became putrid by day 7 as compared to meat with a derived microorganism which did not even start to spoil until day 10.

14

The preferred *Streptococcus lactis* subspecies *diacetilactis* is NRRL-B-15469 with the fusidic acid resistance marker removed by selection and *S. lactis* subspecies *diacetilactis* NRRL-B-15464 with the rifamycin resistance marker removed by selection. Neither of these derived strains are capable of utilizing lactose.

The reference to "NRRL" herein is to the National Regional Research Laboratory in Peoria, Illinois. The designated strains are freely available to those requesting them by reference to the strain name and number.

**Claims**

**Claims for the following Contracting States : BF, CH, DE, FR, GB, IT, LI, LU, NL, SE**

1. A nisin producing derived microorganism which contains foreign DNA, either chromosomal or extrachromosomal, which encodes for nisin production, which foreign DNA was obtained from a nisin producing donor Streptococcus and which derived microorganism is obtainable by mating a nisin-producing donor bacterium with a nisin-sensitive recipient bacterium resulting in the nisin-producing derived microorganism.

2. The nisin producing derived microorganism of Claim 1 wherein the recipient bacterium to which the DNA has been transferred is selected from Streptococcus species, Pediococcus species, Lactobacillus species, Propionibacterium species, Micrococcus species, Leuconostoc species, Staphylococcus species, Clostridium species, Flavobacterium species, Brevibacterium species, E. coli species and Pseudomonas species.

3. The nisin producing derived microorganism of Claim 1 wherein the donor microorganism from which the DNA has been transferred is a nisin producing Streptococcus lactis strain.

4. The nisin producing derived microorganism of Claim 1 wherein the donor microorganism from which the DNA has been transferred is Streptococcus lactis and the recipient microorganism is Streptococcus diacetilactis.

5. The nisin producing derived microorganism of Claim 1 wherein the donor microorganisms from which the DNA has been transferred is selected from Streptococcus lactis ATCC 11,454, NRRL-B-15,458, NRL-B-15,459, NRRL-B-15,460, NRRL-B-15,461, NRRL-B-15,462 and NRRL-B-15,470 and isogenic derivatives thereof.

6. The nisin producing derived microorganism of Claim 1 wherein the recipient bacterium is selected from Streptococcus lactis subspecies diacetilactis NRRL-B-15,005, NRRL-B-15,006, NRRL-B-15,018, NRRL-B-12,070 and NRRL-B-12,071 and isogenic derivatives thereof and/or from Streptococcus lactis NRRL-B-11,452, NRRL-B-15,453 and NRL-B-15,454 and isogenic derivatives thereof.

7. The nisin producing derived microorganism of Claim 1 wherein the recipient bacterium to which the DNA has been transferred produces compounds inhibitory against Gram-negative bacteria, and in particular psychrotrophic bacteria, and the donor Streptococcus from which the DNA has been transferred produces nisin which are both expressed by the derived nisin producing microorganism.

8. The nisin producing derived microorganism of Claim 7 which is inhibitory against Gram-positive bacteria and in particular spore-forming bacteria and also is inhibitory against Gram-negative bacteria and in particular psychrotrophic bacteria.

9. The nisin producing derived microorganism of Claim 7 selected from derived Streptococcus lactis subspecies diacetilactis NRRL-B-15,464, NRRL-B-15,465, NRRL-B-15,466, NRRL-B-15,467, NRRL-B-15,468 and NRRL-B-15,469, and isogenic bacterial derivatives thereof.

10. The nisin producing derived microorganism of any of Claims 1 to 9, wherein the recipient bacterium is non-nisin producing and nisin sensitive, and wherein the introduced DNA is foreign to the recipient bacterium and encodes for a nisin production and is either integrated into the chromosome or is extrachromosomal in the resulting nisin producing microorganism.

**11.** The nisin producing derived microorganism of Claim 10 wherein the recipient bacterium to which the DNA has been transferred is inhibited by nisin and wherein the nisin producing derived microorganism is not inhibited by nisin.

**12.** A method for engineering a nisin producing derived microorganism which comprises:
(a) transferring DNA which DNA encodes for nisin production in a donor Streptococcus to a recipient bacterium which is sensitive to nisin by mating a donor bacterium with a recipient bacterium to result in a nisin producing derived microorganism, wherein the DNA is foreign to the recipient bacterium and encodes for nisin production in the recipient bacterium; and
(b) isolating the nisin producing derived microorganism.

**13.** The method of Claim 12 wherein the recipient bacterium is inhibited by nisin and wherein the isolated nisin producing derived microorganism is not inhibited by nisin.

**14.** The method of claim 12 wherein the recipient bacterium is selected from Streptococcus species, Pediococcus species, Lactobacillus species, Propionibacterium species, Micrococcus species, Leuconostoc species, Staphylococcus species, Clostridium species, Flavobacterium species, Brevibacterium species, E. coli species and Pseudomonas species.

**15.** The method of Claim 12 wherein the donor Streptococcus is selected from Streptococcus lactis ATCC 11,454, NRRL-B-15,458, NRRL-B-15,459, NRRL-B-15,460, NRRL-B-15,461, NRRL-B-15,462, NRRL-B-15,470, and isogenic derivatives thereof.

**16.** The method of Claim 12 wherein the recipient bacterium is selected from Streptococcus lactis subspecies diacetilactis NRRL-B-15,005, NRRL-B-15,006, NRRL-B-15,018 NRRL-B-12,070, NRRL-B-12,071, and isogenic derivatives thereof.

**17.** The method of Claim 12 wherein the derived microorganism is selected from Streptococcus lactis subspecies diacetilactis NRRL-B-15,464, NRRL-B-15,465, NRRL-B-15,466, NRRL-B-15,467, NRRL-B-15,468 and NRRL-B-15,469, and isogenic derivatives thereof.

**18.** In a method of preserving a material by incorporating a microorganism into the material the improvement which comprises incorporating into the material a nisin producing derived microorganism according to any of Claims 1 to 11.

**19.** The method of Claim 18 wherein the material is any substance subject to microbial degradation or spoilage selected from agricultural, industrial and food materials.

**20.** A process for producing nisin, wherein a nisin producing derived microorganism according to any of Claims 1 to 11 is cultivated and nisin is isolated from the culture medium.

**21.** The process according to Claim 20, wherein the isolated nisin is characterized by a bioassay method, which uses nisin sensitive Streptococcus cremoris ATCC 14365.

**22.** A process for producing nisin containing in addition other inhibitory substances, wherein a nisin producing derived microorganism according to any of Claims 1 to 11 is cultivated and nisin and other inhibitory substances are isolated from the culture medium.

**23.** The nisin producing derived microorganism of any of Claims 1 to 11 in a concentrated form, as a liquid non-concentrated culture, concentrated frozen or non-frozen culture, stabilized dried culture and lyophilized culture.

**24.** The nisin producing microorganism of any of Claims 1 to 11 in a non-concentrated form.

**25.** The nisin producing derived microorganism of any of Claims 1 to 11 in a concentrated form which is frozen, non-frozen, non-lyophilized powder, lyophilized powder or stabilized, non-lyophilized dry powder.

**26.** The nisin producing derived microorganism of any of Claims 1 to 11 admixed with a non-nisin producing microorganism.

**27.** The nisin producing derived microorganism of any of Claims 1 to 11 mixed with non-nisin producing Streptococcus lactis subspecies diacetilactis which has been selected for resistance to nisin.

**28.** The nisin producing derived microorganism of any of Claims 1 to 11 as a mixture of nisin-producing derived microorganisms which have different phage susceptibilities so as to enable the assured production of nisin without stringent anti-bacteriophage precautions.

**29.** Use of nisin producing microorganisms according to any of Claims 1 to 11 in the manufacture of a preparation, containing the microorganisms alone or in a mixture with other microorganisms, for use in the treatment of animals.

**30.** An improved method for preservation of a fresh or fermented food product by providing a microorganism in admixture with the food, the improvement which comprises: holding the food at refrigeration temperatures in the presence of the nisin producing derived microorganism of any of Claims 1 to 11.

**31.** A bacterial composition which comprises in admixture:
(a) a nisin producing Streptococcus; and
(b) a nisin resistant Streptococcus diacetilactis of Claim 4 wherein the bacteria in the composition are in biologically pure form.

**32.** The composition of Claim 31 wherein the nisin producing Streptococcus lactis is naturally occurring.

**33.** The composition of Claim 31 in preserved form containing at least about $10^6$ cells per ml.

**34.** The composition of Claim 31 in preserved form containing at least about $10^9$ cells per ml.

**35.** A method for engineering a nisin producing derived microorganism which comprises:
(a) transferring DNA which encodes for nisin production from a nisin producing donor Streptococcus to a recipient bacterium which also produces nisin by mating a donor bacterium with a recipient bacterium which results in a nisin producing derived microorganism which has an enhanced capability for nisin production; and
(b) isolating the derived nisin producing derived microorganism.

**Claims for the following Contracting State : AT**

**1.** A method for engineering a nisin producing derived microorganism which comprises:
(a) transferring DNA which DNA encodes for nisin production in a donor Streptococcus to a recipient bacterium which is sensitive to nisin by mating a donor bacterium with a recipient bacterium to result in a nisin producing derived microorganism, wherein the DNA is foreign to the recipient bacterium and encodes for nisin production in the recipient bacterium; and
(b) isolating the nisin producing derived microorganism.

**2.** The method of Claim 1 wherein the recipient bacterium is inhibited by nisin and wherein the isolated nisin producing derived microorganism is not inhibited by nisin.

**3.** The method of Claim 1 wherein the recipient bacterium is selected from Streptococcus species, Pediococcus species, Lactobacillus species, Propionibacterium species, Micrococcus species, Leuconostoc species, Staphylococcus species, Clostridium species, Flavobacterium species, Brevibacterium species, E. coli species and Pseudomonas species.

**4.** The method of Claim 1 wherein the donor microorganism from which the DNA is transferred is a nisin producing Streptococcus lactis strain.

**5.** The method of Claim 1 wherein the donor Streptococcus is selected from Streptococcus lactis ATCC 11,454, NRRL-B-15,458, NRRL-B-15,459, NRRL-B-15,460, NRRL-B-15,461, NRRL-B-15,462, NRRL-B-

EP 0 137 869 B1

15,470, and isogenic derivatives thereof.

6. The method of Claim 1 wherein the recipient bacterium is selected from Streptococcus lactis subspecies diacetilactis NRRL-B-15,005, NRRL-B-15,006, NRRL-B-15,018 NRRL-B-12,070, NRRL-B-12,071, and isogenic derivatives thereof.

7. The method of Claim 1 wherein the derived microorganism is selected from Streptococcus lactis subspecies diacetilactis NRRL-B-15,464, NRRL-B-15,465, NRRL-B-15,466, NRRL-B-15,467, NRRL-B-15,468 and NRRL-B-15,469, and isogenic derivatives thereof.

8. The method of Claim 1 wherein the recipient bacterium to which the DNA is transferred produces compounds inhibitory against Gram-negative bacteria, and in particular psychrotrophic bacteria, and the donor Streptococcus from which the DNA is transferred produces nisin which are both expressed by the derived nisin producing microorganism.

9. The method of Claim 8 wherein the nisin producing derived microorganism is inhibitory against Gram-positive bacteria and in particular spore-forming bacteria and also is inhibitory against Gram-negative bacteria and in particular psychrotrophic bacteria.

10. The method of any of Claims 1 to 9, wherein the recipient bacterium is non-nisin producing and nisin sensitive, and wherein the DNA to be introduced is foreign to the recipient bacterium and encodes for a nisin production and will be either integrated into the chromosome or will be extrachromosomal in the resulting nisin producing microorganism.

11. The method of Claim 10 wherein the recipient bacterium to which the DNA is to be transferred is inhibited by nisin and wherein the nisin producing derived microorganism is not inhibited by nisin.

12. In a method of preserving a material by incorporating a microorganism into the material the improvement which comprises incorporating into the material a nisin producing derived microorganism produced according to any of Claims 1 to 11.

13. The method of Claim 12 wherein the material is any substance subject to microbial degradation or spoilage selected from agricultural, industrial and food materials.

14. A process for producing nisin, wherein a nisin producing derived microorganism produced according to any of Claims 1 to 11 is cultivated and nisin is isolated from the culture medium.

15. The process according to Claim 14, wherein the isolated nisin is characterized by a bioassay method, which uses nisin sensitive Streptococcus cremoris ATCC 14365.

16. A process for producing nisin containing in addition other inhibitory substances, wherein a nisin producing derived microorganism produced according to any of Claims 1 to 11 is cultivated and nisin and other inhibitory substances are isolated from the culture medium.

17. A process for providing the nising producing derived microorganism produced according to any of Claims 1 to 11 in a concentrated form, as a liquid non-concentrated culture, concentrated frozen or non-frozen culture, stabilized dried culture and lyophilized culture, in a manner known per se.

18. A process for providing the nisin producing derived microorganism produced according to any of Claims 1 to 11 in a non-concentrated form, in a manner known per se.

19. A process for providing the nisin producing derived microorganism produced according to any of Claims 1 to 11 in a concentrated form which is frozen, non-frozen, non-lyophilized powder, lyophilized powder or stabilized, non-lyophilized dry powder, in a manner known per se.

20. A method of preparing a composition wherein the nisin producing derived microorganism produced according to any of Claims 1 to 11 is mixed with a non-nisin producing microorganism.

18

**21.** The method of claim 20 wherein the nisin producing derived microorganism produced according to any of Claims 1 to 11 is mixed with non-nisin producing Streptococcus lactis subspecies diacetilactis which has been selected for resistance to nisin.

**22.** A method of preparing a composition wherein the nisin producing derived microorganism produced according to any of Claims 1 to 11 is mixed with nisin-producing derived microorganisms which have different phage susceptibilities so as to enable the assured production of nisin without stringent anti-bacteriophage precautions.

**23.** Use of nisin producing microorganisms produced according to any of Claims 1 to 11 in the manufacture of a preparation, containing the microorganisms alone or in a mixture with other microorganisms, for use in the treatment of animals.

**24.** An improved method for preservation of a fresh or fermented food product by providing a microorganism in admixture with the food, the improvement which comprises: holding the food at refrigeration temperatures in the presence of the nisin producing derived microorganism produced according to any of Claims 1 to 11.

**25.** A method of preparing a bacterial composition wherein the following components are mixed:
   (a) a nisin producing Streptococcus; and
   (b) a nisin resistant Streptococcus diacetilactis wherein the bacteria in the composition are in biologically pure form.

**26.** The method of Claim 25 wherein the nisin producing Streptococcus lactis is naturally occurring.

**27.** The method of Claim 25 wherein the mixture is preserved and contains at least about $10^6$ cells per ml.

**28.** The method of Claim 25 wherein the mixture is preserved and contains at least about $10^9$ cells per ml.

**29.** A method for engineering a nisin producing derived microorganism which comprises:
   (a) transferring DNA which encodes for nisin production from a nisin producing donor Streptococcus to a recipient bacterium which also produces nisin by mating a donor bacterium with a recipient bacterium which results in a nisin producing derived microorganism which has an enhanced capability for nisin production; and
   (b) isolating the derived nisin producing derived microorganism.

**30.** A nisin producing derived microorganism which contains foreign DNA, either chromosomal or extrachromosomal, which encodes for nisin production, which foreign DNA was obtained from a nisin producing donor Streptococcus and which derived microorganism is obtainable by mating a nisin-producing donor bacterium with a nisin-sensitive recipient bacterium resulting in the nisin-producing derived microorganism.

**31.** The nisin producing derived microorganism of Claim 30 wherein the recipient bacterium to which the DNA has been transferred is selected from Streptococcus species, Pediococcus species, Lactobacillus species, Propionibacterium species, Micrococcus species, Leuconostoc species, Staphylococcus species, Clostridium species, Flavobacterium species, Brevibacterium species, E. coli species and Pseudomonas species.

**32.** The nisin producing derived microorganism of Claim 30 wherein the donor microorganism from which the DNA has been transferred is a nisin producing Streptococcus lactis strain.

**33.** The nisin producing derived microorganism of Claim 30 wherein the donor microorganism from which the DNA has been transferred is Streptococcus lactis and the recipient microorganism is Streptococcus diacetilactis.

**34.** The nisin producing derived microorganism of Claim 30 wherein the donor microorganisms from which the DNA has been transferred is selected from Streptococcus lactis ATCC 11,454, NRRL-B-15,458, NRRL-B-15,459, NRRL-B-15,460, NRRL-B-15,461, NRRL-B-15,462 and NRRL-B-15,470 and isogenic derivatives thereof.

35. The nisin producing derived microorganism of Claim 30 wherein the recipient bacterium is selected from Streptococcus lactis subspecies diacetilactis NRRL-B-15,005, NRRL-B-15,006, NRRL-B-15,018, NRRL-B-12,070 and NRRL-B-12,071 and isogenic derivatives thereof and/or from Streptococcus lactis NRRL-B-11,452, NRRL-B-15,453 and NRRL-B-15,454 and isogenic derivatives thereof.

36. The nisin producing derived microorganism of Claim 30 wherein the recipient bacterium to which the DNA has been transferred produces compounds inhibitory against Gram-negative bacteria, and in particular psychrotrophic bacteria, and the donor Streptococcus from which the DNA has been transferred produces nisin which are both expressed by the derived nisin producing microorganism.

37. The nisin producing derived microorganism of Claim 36 which is inhibitory against Gram-positive bacteria and in particular spore-forming bacteria and also is inhibitory against Gram-negative bacteria and in particular psychrotrophic bacteria.

38. The nisin producing derived microorganism of Claim 36 selected from derived Streptococcus lactis subspecies diacetilactis NRRL-B-15,464, NRRL-B-15,465, NRRL-B-15,466, NRRL-B-15,467, NRRS-B-15,468 and NRRL-B-15,469, and isogenic bacterial derivatives thereof.

39. The nisin producing derived microorganism of any of Claims 30 to 38, wherein the recipient bacterium is non-nisin producing and nisin sensitive, and wherein the introduced DNA is foreign to the recipient bacterium and encodes for a nisin production and is either integrated into the chromosome or is extrachromosomal in the resulting nisin producing microorganism.

40. The nisin producing derived microorganism of Claim 39 wherein the recipient bacterium to which the DNA has been transferred is inhibited by nisin and wherein the nisin producing derived microorganism is not inhibited by nisin.

41. The nisin producing derived microorganism of any of Claims 30 to 40 in a concentrated form, as a liquid non-concentrated culture, concentrated frozen or non-frozen culture, stabilized dried culture and lyophilized culture.

42. The nisin producing microorganism of any of Claims 30 to 40 in a non-concentrated form.

43. The nisin producing derived microorganism of any of Claims 30 to 40 in a concentrated form which is frozen, non-frozen, non-lyophilized powder, lyophilized powder or stabilized, non-lyophilized dry powder.

44. The nisin producing derived microorganism of any of Claims 30 to 40 admixed with a non-nisin producing microorganism.

45. The nisin producing derived microorganism of any of Claims 30 to 40 mixed with non-nisin producing Streptococcus lactis subspecies diacetilactis which has been selected for resistance to nisin.

46. The nisin producing derived microorganism of any of Claims 30 to 40 as a mixture of nisin-producing derived microorganisms which have different phage susceptibilities so as to enable the assured production of nisin without stringent anti-bacteriophage precautions.

47. A bacterial composition which comprises in admixture:
(a) a nisin producing Streptococcus; and
(b) a nisin resistant Streptococcus diacetilactis of Claim 4 wherein the bacteria in the composition are in biologically pure form.

48. The composition of Claim 47 wherein the nisin producing Streptococcus lactis is naturally occurring.

49. The composition of Claim 47 in preserved form containing at least about $10^6$ cells per ml.

20

**50.** The composition of Claim 47 in preserved form containing at least about $10^9$ cells per ml.

**Patentansprüche**
**Patentansprüche für folgende Vertragsstaaten : BE, CH, DE, FR, GB, IT, LI, LU, NL, SE**

**1.** Nisin-herstellender abgeleiteter Mikroorganismus, der Fremd-DNA enthält, entweder chromosomal oder extrachromosomal, welche für Nisinherstellung codiert, wobei die Fremd-DNA von einem Nisin-herstellenden Donor Streptococcus erhalten wurde und wobei der abgeleitete Mikroorganismus erhältlich ist durch Paarung eines Nisin-herstellenden Donor-Bakteriums mit einem Nisin-sensitiven Rezipienten-Bakterium, wobei sich der Nisin-herstellende abgeleitete Mikroorganismus ergibt.

**2.** Nisin-herstellender abgeleiteter Mikroorganismus des Anspruchs 1, worin das Rezipienten-Bakterium, zu welchem die DNA transferiert worden ist, ausgewählt ist aus Streptococcus-Arten, Pediococcus-Arten, Lactobacillus-Arten, Propionibacterium-Arten, Micrococcus-Arten, Leuconostoc-Arten, Staphylococcus-Arten, Clostridium-Arten, Flavobacterium-Arten, Brevibacterium-Arten, E. coli-Arten und Pseudomonas-Arten.

**3.** Nisin-herstellender abgeleiteter Mikroorganismus des Anspruchs 1, worin der Donor-Mikroorganismus, von welchem die DNA transferiert worden ist, ein Nisin-herstellender Streptococcus lactis-Stamm ist.

**4.** Nisin-herstellender abgeleiteter Mikroorganismus des Anspruchs 1, worin der Donor-Mikroorganismus, von welchem die DNA transferiert worden ist, Streptococcus lactis und der Rezipienten-Mikroorganismus Streptococcus diacetilactis ist.

**5.** Nisin-herstellender abgeleiteter Mikroorganismus des Anspruchs 1, worin der Donor-Mikroorganismus, von welchem die DNA transferiert worden ist, ausgewählt ist aus Streptococcus lactis ATCC 11 454, NRRL-B-15 458, NRRL-B-15 459, NRRL-B-15 460, NRRL-B-15 461, NRRL-B-15 462 und NRRL-B-15 470 und isogenen Abkömmlingen davon.

**6.** Nisin-herstellender abgeleiteter Mikroorganismus des Anspruchs 1, worin das Rezipienten-Bakterium ausgewählt ist aus Streptococcus lactis Subspezies diacetilactis NRRL-B-15 005, NRRL-B-15 006, NRRL-B-15 018, NRRL-B-12 070 und NRRL-B-12 071 und isogenen Abkömmlingen davon und/oder aus Streptococcus lactis NRRL-B-11 452, NRRL-B-15 453 und NRRL-B-15 454 und isogenen Abkömmlingen davon.

**7.** Nisin-herstellender abgeleiteter Mikroorganismus des Anspruchs 1, worin das Rezipienten-Bakterium, zu welchem die DNA transferiert worden ist, auf Gram-negative Bakterien, und im besonderen psychrotrophe Bakterien, hemmende Verbindungen herstellt, und der Donor-Streptococcus, von welchem die DNA transferiert worden ist, Nisin herstellt, wobei beides von dem abgeleiteten Nisin-herstellenden Mikroorganismus exprimiert wird.

**8.** Nisin-herstellender abgeleiteter Mikroorganismus des Anspruchs 7, welcher hemmend ist auf Gram-positive Bakterien und im besonderen sporenbildende Bakterien und ebenso hemmend ist auf Gram-negative Bakterien und im besonderen psychrotrophe Bakterien.

**9.** Nisin-herstellender abgeleiteter Mikroorganismus des Anspruchs 7, ausgewählt aus den abgeleiteten Streptococcus lactis Subspezies diacetilactis NRRL-B-15 464, NRRL-B-15 465, NRRL-B-15 466, NRRL-B-15-467, NRRL-B-15 468 und NRRL-B-15 469 und isogenen bakteriellen Abkömmlingen davon.

**10.** Nisin-herstellender abgeleiteter Mikroorganismus nach einem der Ansprüche 1 bis 9, worin das Rezipienten-Bakterium nicht-Nisin-herstellend und Nisin-sensitiv ist, und worin die eingeschleuste DNA für das Rezipienten-Bakterium fremd ist und für Nisinherstellung codiert und entweder in das Chromosom integriert ist oder in dem sich ergebenden Nisin-herstellenden Mikroorganismus extrachromosomal ist.

**11.** Nisin-herstellender abgeleiteter Mikroorganismus des Anspruchs 10, worin das Rezipienten-Bakterium, zu welchem die DNA transferiert worden ist, durch Nisin gehemmt wird und worin der Nisin-herstellende abgeleitete Mikroorganismus nicht durch Nisin gehemmt wird.

EP 0 137 869 B1

**12.** Verfahren zum Erzeugen eines Nisin-herstellenden abgeleiteten Mikroorganismus, welches umfaßt:
a) Transferieren von DNA, die in einem Donor-Streptococcus für Nisinherstellung codiert zu einem Rezipienten-Bakterium, welches gegenüber Nisin sensitiv ist, durch Paarung eines Donor-Bakteriums mit einem Rezipienten-Bakterium, was einen Nisin-herstellenden abgeleiteten Mikroorganismus ergibt, wobei die DNA für das Rezipienten-Bakterium fremd ist und in dem Rezipienten-Bakterium für Nisinherstellung codiert; und
b) Isolieren des Nisin-herstellenden abgeleiteten Mikroorganismus.

**13.** Verfahren des Anspruchs 12, worin das Rezipienten-Bakterium durch Nisin gehemmt wird und worin der isolierte Nisin-herstellende abgeleitete Mikroorganismus nicht durch Nisin gehemmt wird.

**14.** Verfahren des Anspruchs 12, worin das Rezipienten-Bakterium ausgewählt ist aus Streptococcus-Arten, Pediococcus-Arten, Lactobacillus-Arten, Propionibacterium-Arten, Micrococcus-Arten, Leuconostoc-Arten, Staphylococcus-Arten, Clostridium-Arten, Flavobacterium-Arten, Brevibacterium-Arten, E. coli-Arten und Pseudomonas-Arten.

**15.** Verfahren des Anspruchs 12, worin der Donor-Streptococcus ausgewählt ist aus Streptococcus lactis ATCC 11 454, NRRL-B-15 458, NRRL-B-15 459, NRRL-B-15 460, NRRL-B-15 461, NRRL-B-15 462, NRRL-B-15 470 und isogenen Abkömmlingen davon.

**16.** Verfahren des Anspruchs 12, worin das Rezipienten-Bakterium ausgewählt ist aus Streptococcus lactis Subspezies diacetilactis NRRL-B-15 005, NRRL-B-15 006, NRRL-B-15 018, NRRL-B-12 070, NRRL-B-12 071 und isogenen Abkömmlingen davon.

**17.** Verfahren des Anspruchs 12, worin der abgeleitete Mikroorganismus ausgewählt ist aus Streptococcus lactis Subspezies diacetilactis NRRL-B-15 464, NRRL-B-15 465, NRRL-B-15 466, NRRL-B-15 467, NRRL-B-15 468 und NRRL-B-15 469 und isogenen Abkömmlingen davon.

**18.** In einem Verfahren zum Konservieren eines Materials durch Einbringen eines Mikroorganismus in das Material die Verbesserung, welche umfaßt: Einbringen eines Nisin-herstellenden abgeleiteten Mikroorganismus gemäß einem der Ansprüche 1 bis 11 in das Material.

**19.** Verfahren des Anspruchs 18, worin das Material jede Substanz ist, die mikrobiellem Abbau oder Fäulnis ausgesetzt ist, ausgewählt aus landwirtschaftlichen, industriellen und Nahrungsmaterialien.

**20.** Verfahren zum Herstellen von Nisin, worin ein Nisin-herstellender abgeleiteter Mikroorganismus gemäß einem der Ansprüche 1 bis 11 kultiviert wird und Nisin aus dem Kulturmedium isoliert wird.

**21.** Verfahren nach Anspruch 20, worin das isolierte Nisin durch ein Biotest-Verfahren charakterisiert wird, welches Nisin-sensitiven Streptococcus cremoris ATCC 14365 verwendet.

**22.** Verfahren zum Herstellen von Nisin, enthaltend zusätzlich andere hemmende Substanzen, worin ein Nisin-herstellender abgeleiteter Mikroorganismus gemäß einem der Ansprüche 1 bis 11 kultiviert wird und Nisin und andere hemmende Substanzen aus dem Kulturmedium isoliert werden.

**23.** Nisin-herstellender abgeleiteter Organismus nach einem der Ansprüche 1 bis 11 in einer konzentrierten Form, als flüssige nicht-konzentrierte Kultur, konzentrierte gefrorene oder nicht-gefrorene Kultur, stabilisierte getrocknete Kultur und lyophilisierte Kultur.

**24.** Nisin-herstellender Mikroorganisums nach einem der Ansprüche 1 bis 11 in einer nicht-konzentrierten Form.

**25.** Nisin-herstellender abgeleiteter Mikroorganismus nach einem der Ansprüche 1 bis 11 in einer konzentrierten Form, welche gefroren, nicht-gefroren, nicht-lyophilisiertes Pulver, lyophilisiertes Pulver oder stabilisiertes, nicht-lyophilisiertes Trockenpulver ist.

**26.** Nisin-herstellender abgeleiteter Mikroorganismus nach einem der Ansprüche 1 bis 11, gemischt mit einem nicht-Nisin-herstellenden Mikroorganismus.

22

**27.** Nisin-herstellender abgeleiteter Mikroorganismus nach einem der Ansprüche 1 bis 11, gemischt mit nicht-Nisin-herstellendem Streptococcus lactis Subspezies diacetilactis, welcher auf Resistenz gegenüber Nisin selektiert worden ist.

**28.** Nisin-herstellender abgeleiteter Mikroorganismus nach einem der Ansprüche 1 bis 11 als eine Mischung Nisin-herstellender abgeleiteter Mikroorganismen, die verschiedene Phagenanfälligkeit besitzen, so daß eine gesicherte Herstellung von Nisin ohne strenge Maßnahmen gegen Bakteriophagen ermöglicht wird.

**29.** Verwendung Nisin-herstellender Mikroorganismen gemäß einem der Ansprüche 1 bis 11 für die Herstellung einer Zubereitung, enthaltend die Mikroorganismen allein oder in Mischung mit anderen Mikroorganismen zur Verwendung bei der Behandlung von Tieren.

**30.** Verbessertes Verfahren zur Konservierung eines frischen oder fermentierten Nahrungsmittelproduktes durch Bereitstellen eines Mikroorganismus in Mischung mit dem Nahrungsmittel, wobei die Verbesserung umfaßt:
Halten des Nahrungsmittels bei Kühltemperaturen im Vorhandensein des Nisin-herstellenden abgeleiteten Mikroorganismus nach einem der Ansprüche 1 bis 11.

**31.** Bakterielle Zusammensetzung, welche in Mischung umfaßt:
a) einen Nisin-herstellenden Streptococcus; und
b) einen Nisin-resistenten Streptococcus diacetilactis des Anspruchs 4, worin die Bakterien in der Zusammensetzung in biologisch reiner Form sind.

**32.** Zusammensetzung des Anspruchs 31, worin der Nisin-herstellende Streptococcus lactis natürlich vorkommt.

**33.** Zusammensetzung des Anspruchs 31 in haltbar gemachter Form, enthaltend wenigstens ungefähr $10^6$ Zellen pro ml.

**34.** Zusammensetzung des Anspruchs 31 in haltbar gemachter Form, enthaltend wenigstens ungefähr $10^9$ Zellen pro ml.

**35.** Verfahren zum Erzeugen eines Nisin-herstellenden abgeleiteten Mikroorganismus, welches umfaßt:
a) Transferieren von DNA, welche für Nisinherstellung codiert von einem Nisin-herstellenden Donor-Streptococcus zu einem Rezipienten-Bakterium, welches ebenso Nisin produziert durch Paarung eines Donor-Bakteriums mit einem Rezipienten-Bakterium, was einen Nisin-herstellenden abgeleiteten Mikroorganismus ergibt, welcher eine gesteigerte Fähigkeit zur Nisinherstellung hat; und
b) Isolieren des abgeleiteten Nisin-herstellenden abgeleiteten Mikroorganismus.

**Patentansprüche für folgenden Vertragsstaat : AT**

**1.** Verfahren zum Erzeugen eines Nisin-herstellenden abgeleiteten Mikroorganismus, welches umfaßt:
a) Transferieren von DNA, welche in einem Donor-Streptococcus für Nisinherstellung codiert zu einem Rezipienten-Bakterium, welches gegenüber Nisin sensitiv ist durch Paaren eines Donor-Bakteriums mit einem Rezipienten-Bakterium, um einen Nisin-herstellenden abgeleiteten Mikroorganismus zu erzeugen, worin die DNA für das Rezipienten-Bakterium fremd ist und in dem Rezipienten-Bakterium für Nisinherstellung codiert; und
b) Isolieren des Nisin-herstellenden abgeleiteten Mikroorganismus.

**2.** Verfahren des Anspruchs 1, worin das Rezipienten-Bakterium durch Nisin gehemmt wird und worin der isolierte Nisin-herstellende abgeleitete Mikroorganismus nicht durch Nisin gehemmt wird.

**3.** Verfahren des Anspruchs 1, worin das Rezipienten-Bakterium ausgewählt ist aus Streptococcus-Arten, Pediococcus-Arten, Lactobacillus-Arten, Propionibacterium-Arten, Micrococcus-Arten, Leuconostoc-Arten, Staphylococcus-Arten, Clostridium-Arten, Flavobacterium-Arten, Brevibacterium-Arten, E. coli-Arten und Pseudomonas-Arten.

23

**4.** Verfahren des Anspruchs 1, worin der Donor-Mikroorganismus, von welchem die DNA transferiert wird, ein Nisin-herstellender Streptococcus lactis-Stamm ist.

**5.** Verfahren des Anspruchs 1, worin der Donor-Streptococcus ausgewählt ist aus Streptococcus lactis ATCC 11 454, NRRL-B-15 458, NRRL-B-15 459, NRRL-B-15 460, NRRL-B-15 461, NRRL-B-15 462, NRRL-B-15 470 und isogenen Abkömmlingen davon.

**6.** Verfahren des Anspruchs 1, worin das Rezipienten-Bakterium ausgewählt ist aus Streptococcus lactis Subspezies diacetilactis NRRL-B-15 005, NRRL-B-15 006 NRRL-B-15 018, NRRL-B-12 070, NRRL-B-12 071 und isogenen Abkömmlingen davon.

**7.** Verfahren des Anspruchs 1, worin der abgeleitete Mikroorganismus ausgewählt ist aus Streptococcus lactis Subspezies diacetilactis NRRL-B-15 464, NRRL-B-15 465, NRRL-B-15 466, NRRL-B-15 467, NRRL-B-15 468 und NRRL-B-15 469 und isogenen Abkömmlingen davon.

**8.** Verfahren des Anspruchs 1, worin das Rezipienten-Bakterium, zu welchem die DNA transferiert wird, auf Gram-negative Bakterien, und im besonderen psychrotrophe Bakterien, hemmende Verbindungen herstellt, und der Donor-Streptococcus, von welchem die DNA transferiert wird, Nisin herstellt, wobei beides von dem abgeleiteten Nisin-herstellenden Mikroorganismus exprimiert wird.

**9.** Verfahren des Anspruchs 8, worin der Nisin-herstellende abgeleitete Mikroorganismus auf Gram-positive Bakterien und im besonderen sporenbildende Bakterien hemmend ist und ebenso auf Gram-negative Bakterien und im besonderen psychrotrophe Bakterien hemmend ist.

**10.** Verfahren nach einem der Ansprüche 1 bis 9, worin das Rezipienten-Bakterium nicht-Nisin-herstellend und Nisin-sensitiv ist, und worin die einzuschleusende DNA für das Rezipienten-Bakterium fremd ist und für eine Nisinherstellung codiert und entweder in das Chromosom integriert wird oder in dem sich ergebenden Nisin-herstellenden Mikroorganismus extrachromosomal sein wird.

**11.** Verfahren des Anspruchs 10, worin das Rezipienten-Bakterium, zu welchem die DNA transferiert werden soll, durch Nisin gehemmt wird und worin der Nisin-herstellende abgeleitete Mikroorganismus nicht durch Nisin gehemmt wird.

**12.** In einem Verfahren zum Konservieren eines Materials durch Einbringen eines Mikroorganismus in das Material die Verbesserung, welche umfaßt: Einbringen eines Nisin-herstellenden abgeleiteten Mikroorganismus, der gemäß einem der Ansprüche 1 bis 11 hergestellt wurde, in das Material.

**13.** Verfahren des Anspruchs 12, worin das Material jede Substanz ist, die einem mikrobiellen Abbau oder Fäulnis ausgesetzt ist, ausgewählt aus landwirtschaftlichen, industriellen und Nahrungsmaterialien.

**14.** Verfahren zum Herstellen von Nisin, worin ein Nisin-herstellender abgeleiteter Mikroorganismus, hergestellt gemäß einem der Ansprüche 1 bis 11, kultiviert wird und Nisin aus dem Kulturmedium isoliert wird.

**15.** Verfahren gemäß Anspruch 14, worin das isolierte Nisin durch ein Biotest-Verfahren charakterisiert wird, welches den Nisin-sensitiven Streptococcus cremoris ATCC 14365 verwendet.

**16.** Verfahren zum Herstellen von Nisin, enthaltend zusätzlich andere hemmende Substanzen, worin ein Nisin-herstellender abgeleiteter Mikroorganismus, hergestellt gemäß einem der Ansprüche 1 bis 11, kultiviert wird und Nisin und andere hemmende Substanzen aus dem Kulturmedium isoliert werden.

**17.** Verfahren zum Bereitstellen des Nisin-herstellenden abgeleiteten Mikroorganismus, hergestellt gemäß einem der Ansprüche 1 bis 11, in einer konzentrierten Form, als eine flüssige nicht-konzentrierte Kultur, konzentrierte gefrorene oder nicht-gefrorene Kultur, stabilisierte getrocknete Kultur und lyophilisierte Kultur, auf an sich bekannte Weise.

**18.** Verfahren zum Bereitstellen des Nisin-herstellenden abgeleiteten Mikroorganismus, hergestellt gemäß einem der Ansprüche 1 bis 11, in einer nicht-konzentrierten Form auf an sich bekannte Weise.

24

EP 0 137 869 B1

**19.** Verfahren zum Bereitstellen des Nisin-herstellenden abgeleiteten Mikroorganismus, hergestellt gemäß einem der Ansprüche 1 bis 11 in einer konzentrierten Form, die gefroren, nicht-gefroren, nicht-lyophilisiertes Pulver, lyophilisiertes Pulver oder stabilisiertes nicht-lyophilisiertes Trockenpulver ist auf an sich bekannte Weise.

**20.** Verfahren zum Herstellen einer Zusammensetzung, worin der Nisin-herstellende abgeleitete Mikroorganismus, hergestellt gemäß einem der Ansprüche 1 bis 11, gemischt wird mit einem nicht-Nisin-herstellenden Mikroorganismus.

**21.** Verfahren des Anspruchs 20, worin der Nisin-herstellende abgeleitete Mikroorganismus, hergestellt gemäß einem der Ansprüche 1 bis 11, gemischt wird mit nicht-Nisin-herstellendem Streptococcus lactis Subspezies diacetilactis, der auf Resistenz gegenüber Nisin selektiert worden ist.

**22.** Verfahren zum Herstellen einer Zusammensetzung, worin der Nisin-herstellende abgeleitete Mikroorganismus, hergestellt gemäß einem der Ansprüche 1 bis 11, gemischt wird mit Nisin-herstellenden abgeleiteten Mikroorganismen, welche verschiedene Phagenanfälligkeit besitzen, so daß die gesicherte Herstellung von Nisin ohne strenge Maßnahmen gegen Bakteriophagen ermöglicht wird.

**23.** Verwendung der Nisin-herstellenden Mikroorganismen, hergestellt gemäß einem der Ansprüche 1 bis 11 für die Herstellung einer Zubereitung, enthaltend die Mikroorganismen allein oder in Mischung mit anderen Mikroorganismen, zur Verwendung bei der Behandlung von Tieren.

**24.** Verbessertes Verfahren zum Konservieren eines frischen oder fermentierten Nahrungsmittelproduktes durch Bereitstellen eines Mikroorganismus in Mischung mit dem Nahrungsmittel, wobei die Verbesserung umfaßt: Halten des Nahrungsmittels bei Kühltemperaturen im Vorhandensein des Nisin-herstellenden abgeleiteten Mikroorganismus, hergestellt gemäß einem der Ansprüche 1 bis 11.

**25.** Verfahren zum Herstellen einer bakteriellen Zusammensetzung, worin die folgenden Komponenten gemischt werden:
a) ein Nisin-herstellender Streptococcus; und
b) ein Nisin-resistenter Streptococcus diacetilactis, worin die Bakterien in der Zusammensetzung in biologisch reiner Form sind.

**26.** Verfahren des Anspruchs 25, worin der Nisin-herstellende Streptococcus lactis natürlich vorkommt.

**27.** Verfahren des Anspruchs 25, worin die Mischung haltbar gemacht ist und wenigstens ungefähr $10^6$ Zellen pro ml enthält.

**28.** Verfahren des Anspruchs 25, worin die Mischung haltbar gemacht ist und mindestens ungefähr $10^9$ Zellen pro ml enthält.

**29.** Verfahren zum Erzeugen eines Nisin-herstellenden abgeleiteten Mikroorganismus, welches umfaßt:
a) Transferieren von DNA, welche für Nisinherstellung codiert von einem Nisin-herstellenden Donor-Streptococcus zu einem Rezipienten-Bakterium, welches ebenso Nisin herstellt durch Paaren eines Donor-Bakteriums mit einem Rezipienten-Bakterium, was einen Nisin-herstellenden abgeleiteten Mikroorganismus ergibt, welcher eine gesteigerte Fähigkeit zur Nisinherstellung besitzt; und
b) Isolieren des abgeleiteten Nisin-herstellenden abgeleiteten Mikroorganismus.

**30.** Nisin-herstellender abgeleiteter Mikroorganismus, welcher Fremd-DNA enthält, entweder chromosomal oder extrachromosomal, welche für Nisinherstellung codiert, wobei die Fremd-DNA von einem Nisin-herstellenden Donor-Streptococcus erhalten wurde und wobei der abgeleitete Mikroorganismus erhältlich ist durch Paarung eines Nisin-herstellenden Donor-Bakteriums mit einem Nisin-sensitiven Rezipienten-Bakterium, wobei sich der Nisin-herstellende abgeleitete Mikroorganismus ergibt.

**31.** Nisin-herstellender abgeleiteter Mikroorganismus des Anspruchs 30, worin das Rezipienten-Bakterium, zu welchem die DNA transferiert worden ist, ausgewählt ist aus Streptococcus-Arten, Pediococcus-Arten, Lactobacillus-Arten, Propionbacterium-Arten, Micrococcus-Arten, Leuconostoc-Arten, Staphylococcus-Arten, Clostridium-Arten, Flavobacterium-Arten, Brevibacterium-Arten, E. coli-Arten und Pseudo-

25

monas-Arten.

**32.** Nisin-herstellender abgeleiteter Mikroorganismus des Anspruchs 30, worin der Donor-Mikroorganismus, von welchem die DNA transferiert worden ist, ein Nisin-herstellender Streptococcus lactis-Stamm ist.

**33.** Nisin-herstellender abgeleiteter Mikroorganismus des Anspruchs 30, worin der Donor-Mikroorganismus, von welchem die DNA transferiert worden ist, Streptococcus lactis ist und der Rezipienten-Mikroorganismus Streptococcus diacetilactis ist.

**34.** Nisin-herstellender abgeleiteter Mikroorganismus des Anspruchs 30, worin der Donor-Mikroorganismus, von welchem die DNA transferiert worden ist, ausgewählt ist aus Streptococcus lactis ATCC 11 454, NRRL-B-15 458, NRRL-B-15 459, NRRL-B-15 460, NRRL-B-15 461, NRRL-B-15 462 und NRRL-B-15 470 und isogenen Abkömmlingen davon.

**35.** Nisin-herstellender abgeleiteter Mikroorganismus des Anspruchs 30, worin das Rezipienten-Bakterium ausgewählt ist aus Streptococcus lactis Subspezies diacetilactis NRRL-B-15 005, NRRL-B-15 006, NRRL-B-15 018, NRRL-B-12 070 und NRRL-B-12 071 und isogenen Abkömmlingen davon und/oder von Streptococcus lactis NRRL-B-11 452, NRRL-B-15 453 und NRRL-B-15 454 und isogenen Abkömmlingen davon.

**36.** Nisin-herstellender abgeleiteter Mikroorganismus des Anspruchs 30, worin das Rezipienten-Bakterium, zu welchem die DNA transferiert worden ist, auf Gram-negative Bakterien, und im besonderen psychrotrophe Bakterien, hemmende Verbindungen herstellt, und der Donor-Streptococcus, von welchem die DNA transferiert worden ist, Nisin herstellt, wobei beides durch den abgeleiteten Nisin-herstellenden Mikroorganismus exprimiert wird.

**37.** Nisin-herstellender abgeleiteter Mikroorganismus des Anspruchs 36, welcher auf Gram-negative Bakterien und im besonderen sporenbildenden Bakterium hemmend ist und ebenso auf Gram-negative Bakterien und im besonderen psychrotrophe Bakterien hemmend ist.

**38.** Nisin-herstellender abgeleiteter Mikroorganismus des Anspruchs 36, ausgewählt aus den abgeleiteten Streptococcus lactis Subspezies diacetilactis NRRL-B-15 464, NRRL-B-15 465, NRRL-B-15 466, NRRL-B-15 467, NRRL-B-15 468 und NRRL-B-15 469 und isogenen bakteriellen Abkömmlingen davon.

**39.** Nisin-herstellender abgeleiteter Mikroorganismus nach einem der Ansprüche 30 bis 38, worin das Rezipienten-Bakterium nicht-Nisin-herstellend und Nisin-sensitiv ist, und worin die eingeschleuste DNA für das Rezipienten-Bakterium fremd ist und für eine Nisinherstellung codiert und entweder in das Chromosom integriert ist oder in dem sich ergebenden Nisin-herstellenden Mikroorganismus extrachromosomal ist.

**40.** Nisin-herstellender abgeleiteter Mikroorganismus des Anspruchs 39, worin das Rezipienten-Bakterium, zu welchem die DNA transferiert worden ist, durch Nisin gehemmt wird und worin der Nisin-herstellende abgeleitete Mikroorganismus nicht durch Nisin gehemmt wird.

**41.** Nisin-herstellender abgeleiteter Mikroorganismus nach einem der Ansprüche 30 bis 40 in einer konzentrierten Form, als eine flüssige nicht-konzentrierte Kultur, konzentrierte gefrorene oder nicht-gefrorene Kultur, stabilisierte getrocknete Kultur und lyophilisierte Kultur.

**42.** Nisin-herstellender Mikroorganismus nach einem der Ansprüche 30 bis 40 in einer nicht-konzentrierten Form.

**43.** Nisin-herstellender abgeleiteter Mikroorganismus nach einem der Ansprüche 30 bis 40 in einer konzentrierten Form, welche gefroren, nicht-gefroren, nicht-lyophilisiertes Pulver, lyophilisiertes Pulver oder stabilisiertes, nicht-lyophilisiertes Trockenpulver ist.

**44.** Nisin-herstellender abgeleiteter Mikroorganismus nach einem der Ansprüche 30 bis 40 gemischt mit einem nicht-Nisin-herstellenden Mikroorganismus.

26

**45.** Nisin-herstellender abgeleiteter Mikroorganismus nach einem der Ansprüche 30 bis 40 gemischt mit nicht-Nisin-herstellendem Streptococcus lactis Subspezies diacetilactis, welcher auf Resistenz gegenüber Nisin selektiert worden ist.

**46.** Nisin-herstellender abgeleiteter Mikroorganismus nach einem der Ansprüche 30 bis 40 als Mischung von Nisin-herstellenden abgeleiteten Mikroorganismen, die verschiedene Phagenanfälligkeit besitzen, so daß die gesicherte Herstellung von Nisin ohne strenge Maßnahmen gegen Bakteriophagen ermöglicht wird.

**47.** Bakterielle Zusammensetzung, welche in Mischung umfaßt:
a) einen Nisin-herstellenden Streptococcus; und
b) einen Nisin-resistenten Streptococcus diacetilactis des Anspruchs 4, worin die Bakterien in der Zusammensetzung in einer biologisch reinen Form sind.

**48.** Zusammensetzung des Anspruchs 47, worin der Nisin-herstellende Streptococcus lactis natürlich vorkommt.

**49.** Zusammensetzung des Anspruchs 47 in haltbar gemachter Form, enthaltend wenigstens ungefähr $10^6$ Zellen pro ml.

**50.** Zusammensetzung des Anspruchs 47 in haltbar gemachter Form, enthaltend wenigstens ungefähr $10^9$ Zellen pro ml.

**Revendications**

**Revendications pour les Etats contractants suivants : BE, CH, DE, FR, GB, IT, LI, LU, NL, SE**

**1.** Micro-organisme modifié producteur de nisine, contenant un ADN étranger chromosomique ou extra-chromosomique, qui code pour la production de nisine, cet ADN étranger étant obtenu à partir d'un Streptococcus donneur producteur de nisine, ce micro-organisme modifié pouvant être obtenu par conjugaison d'une bactérie donneuse productrice de nisine avec une bactérie receveuse sensible à la nisine, résultant en le micro-organisme modifié producteur de nisine.

**2.** Micro-organisme modifié producteur de nisine selon la revendication 1, la bactérie receveuse dans laquelle l'ADN a été transféré, étant choisie parmi les espèces des genres Streptococcus, Pediococcus, Lactobacillus, Propionibacterium, Micrococcus, Leuconostoc, Staphylococcus, Clostridium, Flavobacterium, Brevibacterium, l'espèce E. coli et les espèces du genre Pseudomonas.

**3.** Micro-organisme modifié producteur de nisine selon la revendication 1, le micro-organisme donneur à partir duquel l'ADN a été transféré, étant une souche de Streptococcus lactis productrice de nisine.

**4.** Micro-organisme modifié producteur de nisine selon la revendication 1, le micro-organisme donneur à partir duquel l'ADN a été transféré, étant Streptococcus lactis, et le micro-organisme receveur étant Streptococcus diacetilactis.

**5.** Micro-organisme modifié producteur de nisine selon la revendication 1, les micro-organismes donneurs à partir desquels l'ADN a été transféré, étant choisis parmi Streptococcus lactis ATCC 11 454, NRRL-B-15 458, NRRL-B-15 459, NRRL-B-15 460, NRRL-B-15 461, NRRL-B-15 462 et NRRL-B-15 470, et leurs dérivés isogéniques.

**6.** Micro-organisme modifié producteur de nisine selon la revendication 1, la bactérie receveuse étant choisie parmi Streptococcus lactis de la sous-espèce diacetilactis NRRL-B-15 005, NRRL-B-15 006, NRRL-B-15018, NRRL-B-12 070 et NRRS-B-12 071, et leurs dérivés isogéniques et/ou parmi Streptococcus lactis NRRL-B-11 452, NRRL-B-15 453 et NRRL-B-15 454, et leurs dérivés isogéniques.

**7.** Micro-organisme modifié producteur de nisine selon la revendication 1, la bactérie receveuse dans laquelle l'ADN a été transféré, produisant des composés inhibiteurs de bactéries Gram-négatif, et en particulier les bactéries psychotropes, et le Streptococcus donneur à partir duquel l'ADN a été transféré, produisant de la nisine, ces deux produits étant formés par le micro-organisme producteur de nisine.

**8.** Micro-organisme modifié producteur de nisine selon la revendication 7, qui inhibe les bactéries Gram-positif et en particulier les bactéries sporogènes, et qui inhibe également les bactéries Gram-négatif, en particulier les bactéries psychotropes.

**9.** Micro-organisme modifié producteur de nisine selon la revendication 7, choisi parmi les Stroptococcus lactis de la sous-espèce diacetilactis modifiés NRRL-B-15 464, NRRL-B-15 465, NRRL-B-15 466, NRRL-B-15 467, NRRL-B-15 468 et NRRL-B-15 469, et leurs dérivés bactériens isogéniques.

**10.** Micro-organisme modifié producteur de nisine selon l'une quelconque des revendications 1 à 9, la bactérie receveuse étant non productrice de nisine et sensible à la nisine, et l'ADN introduit étant étranger à la bactérie receveuse, et codant pour une production de nisine, et il est intégré dans le chromosome ou à l'extérieur du chromosone dans le micro-organisme producteur de nisine résultant.

**11.** Micro-organisme modifié producteur de nisine selon la revendication 10, la bactérie receveuse dans laquelle l'ADN a été transféré, étant inhibée par la nisine, et le micro-organisme modifié producteur de nisine, n'étant pas inhibé par la nisine.

**12.** Procédé d'élaboration d'un micro-organisme modifié producteur de nisine, selon lequel :
(a) on transfère un ADN, cet ADN codant pour la production de nisine dans un Streptococcus donneur, à une bactérie receveuse sensible à la nisine, par conjugaison d'une bactérie donneuse avec une bactérie receveuse afin de former un micro-organisme modifié producteur de nisine, l'ADN étant étranger à la bactérie receveuse et codant pour la production de nisine dans la bactérie receveuse ; et
(b) on isole le micro-organisme modifié producteur de nisine.

**13.** Procédé selon la revendication 12, dans lequel la bactérie receveuse est inhibée par la nisine, et dans lequel le micro-organisme modifié producteur de nisine isolé, n'est pas inhibé par la nisine.

**14.** Procédé selon la revendication 12, dans lequel la bactérie receveuse est choisie parmi les espèces des genres Streptococcus, Pediococcus, Lactobacillus, Propionibacterium, Micrococcus, Leuconostoc, Staphylococcus, Clostridium, Flavobacterium, Brevibacterium, l'espèce E. coli et les espèces du genre Pseudomonas.

**15.** Procédé selon la revendication 12, dans lequel le Streptococcus donneur est choisi parmi Streptococcus lactis ATCC 11 454, NRRL-B-15 458, NRRL-B-15 459, NRRL-B-15 460, NRRL-B-15 461, NRRL-B-15 462, NRRL-B-15 470 et leurs dérivés isogéniques.

**16.** Procédé selon la revendication 12, dans lequel la bactérie receveuse est choisie parmi Streptococcus lactis de la sous-espèce diacetilactis NRRL-B-15 005, NRRL-B-15 006, NRRL-B-15 018, NRRL-B-12 070, NRRL-B-12 071, et leurs dérivés isogéniques.

**17.** Procédé selon la revendication 12, dans lequel le micro-organisme modifié est choisi parmi Streptococcus lactis de la sous-espèce diacetilactis NRRL-B-15 464, NRRL-B-15 465, NRRL-B-15 466, NRRL-B-15 467, NRRL-B-15 468 et NRRL-B-15 469, et leurs dérivés isogéniques.

**18.** Procédé de conservation d'une matière en incorporant un micro-organisme dans la matière, caractérisé en ce qu'on incorpore dans la matière, un micro-organisme modifié producteur de nisine, selon l'une quelconque des revendications 1 à 11.

**19.** Procédé selon la revendication 18, dans lequel la matière est une quelconque substance sujette à une dégradation ou une altération, choisie parmi les matières agricoles, industrielles et alimentaires.

**20.** Procédé de production de nisine, dans lequel on cultive un micro-organisme modifié producteur de nisine selon l'une quelconque des revendications 1 à 11, et on isole la nisine à partir du milieu de culture.

**21.** Procédé selon la revendication 20, dans lequel la nisine isolée, est caractérisée selon un procédé d'analyse biologique mettant en oeuvre Streptococcus cremoris ATCC 14365 sensible à la nisine.

**22.** Procédé de production de nisine contenant en outre d'autres substances inhibitrices, dans lequel on cultive un micro-organisme modifié producteur de nisine selon l'une quelconque des revendications 1 à 11, et on isole du milieu du culture, la nisine et les autres substances inhibitrices.

**23.** Micro-organisme modifié producteur de nisine selon l'une quelconque des revendications 1 à 11, sous forme concentrée, d'une culture liquide non concentrée, d'une culture concentrée congelée ou non congelée, d'une culture séchée et stabilisée et d'une culture lyophilisée.

**24.** Micro-organisme producteur de nisine selon l'une quelconque des revendications 1 à 11, sous forme non concentrée.

**25.** Micro-organisme modifié producteur de nisine selon l'une quelconque des revendications 1 à 11, sous forme concentrée, comprenant une poudre non lyophilisée et congelée ou non congelée, une poudre lyophilisée ou une poudre sèche non lyophilisée et stabilisée.

**26.** Micro-organisme modifié producteur de nisine selon l'une quelconque des revendications 1 à 11, mélangé avec un micro-organisme non producteur de nisine.

**27.** Micro-organisme modifié producteur de nisine selon l'une quelconque des revendications 1 à 11, mélangé avec Streptococcus lactis de la sous-espèce diacetilactis non producteur de nisine, ayant été sélectionné eu égard à la résistance à la nisine.

**28.** Micro-organisme modifié producteur de nisine selon l'une quelconque des revendications 1 à 11, mélangé avec des micro-organismes modifiés producteurs de nisine, ayant différentes sensibilités aux phages, de façon à permettre la production assurée de nisine sans précautions rigoureuses contre les bactériophages.

**29.** Utilisation de micro-organismes producteurs de nisine selon l'une quelconque des revendications 1 à 11, pour la préparation d'une composition contenant les micro-organismes seuls ou en mélange avec d'autres micro-organismes, destinée à une utilisation dans le traitement d'animaux.

**30.** Procédé amélioré de conservation d'un produit alimentaire frais ou fermenté, en mélangeant un micro-organisme avec l'aliment, caractérisé en ce qu'on maintient l'aliment à des températures de réfrigération, en présence du micro-organisme modifié producteur de nisine selon l'une quelconque des revendications 1 à 11.

**31.** Composition bactéricide, comprenant en mélange :
(a) un Streptococcus producteur de nisine ; et
(b) un Streptococcus diacetilactis résistant à la nisine selon la revendication 4, les bactéries incorporées dans la composition étant sous forme biologiquement pure.

**32.** Composition selon la revendication 31, dans laquelle le Streptococcus lactis producteur de nisine, est naturel.

**33.** Composition selon la revendication 31, sous forme préservée contenant au moins environ $10^6$ cellules par ml.

**34.** Composition selon la revendication 31, sous forme préservée contenant au moins environ $10^9$ cellules par ml.

**35.** Procédé d'élaboration d'un micro-organisme modifié producteur de nisine, selon lequel :
(a) on transfère un ADN qui code pour la production de nisine à partir d'un Streptococcus donneur producteur de nisine, à une bactérie receveuse qui produit également de la nisine, par conjugaison d'une bactérie donneuse avec une bactérie receveuse, résultant en un micro-organisme modifié producteur de nisine ayant une capacité de production de nisine accrue ; et

(b) on isole le micro-organisme modifié producteur de nisine obtenu.

**Revendications pour l'Etat contractant suivant : AT**

1. Procédé d'élaboration d'un micro-organisme modifié producteur de nisine, selon lequel :
   (a) on transfère un ADN, cet ADN codant pour la production de nisine dans un Streptococcus donneur, à une bactérie receveuse sensible à la nisine, par conjugaison d'une bactérie donneuse avec une bactérie receveuse afin de former un micro-organisme modifié producteur de nisine, l'ADN étant étranger à la bactérie receveuse et codant pour la production de nisine dans la bactérie receveuse ; et
   (b) on isole le micro-organisme modifié producteur de nisine.

2. Procédé selon la revendication 1, dans lequel la bactérie receveuse est inhibée par la nisine, et dans lequel le micro-organisme modifié producteur de nisine isolé, n'est pas inhibé par la nisine.

3. Procédé selon la revendication 1, dans lequel la bactérie receveuse est choisie parmi les espèces des genres Streptococcus, Pediococcus, Lactobacillus, Propionibacterium, Micrococcus, Leuconostoc, Staphylococcus, Clostridium, Flavobacterium, Brevibacterium, l'espèce E. coli et les espèces du genre Pseudomonas.

4. Procédé selon la revendication 1, dans lequel le micro-organisme donneur à partir duquel l'ADN a été transféré, est une souche de Streptococcus lactis productrice de nisine.

5. Procédé selon la revendication 1, dans lequel le Streptococcus donneur est choisi parmi Streptococcus lactis ATCC 11 454, NRRL-B-15 458, NRRL-B-15 459, NRRL-B-15 460, NRRL-B-15 461, NRRL-B-15 462, NRRL-B-15 470 et leurs dérivés isogéniques.

6. Procédé selon la revendication 1, dans lequel la bactérie receveuse est choisie parmi Streptococcus lactis de la sous-espèce diacetilactis NRRL-B-15 005, NRRL-B-15 006, NRRL-B-15 018, NRRL-B-12 070, NRRL-B-12 071, et leurs dérivés isogéniques.

7. Procédé selon la revendication 1, dans lequel le micro-organisme modifié est choisi parmi Streptococcus lactis de la sous-espèce diacetilactis NRRL-B-15 464, NRRL-B-15 465, NRRL-B-15 466, NRRL-B-15 467, NRRL-B-15 468 et NRRL-B-15 469, et leurs dérivés isogéniques.

8. Procédé selon la revendication 1, dans lequel la bactérie receveuse dans laquelle l'ADN est transféré, produit des composés inhibiteurs des bactéries Gram-négatif, et en particulier les bactéries psychotropes, et dans lequel le Streptococcus donneur à partir duquel l'ADN a été transféré, produit de la nisine, ces deux produits étant formés par le micro-organisme producteur de nisine modifié.

9. Procédé selon la revendication 8, dans lequel le micro-organisme modifié producteur de nisine, inhibe les bactéries Gram-positif, et en particulier les bactéries sporogènes, et il inhibe également les bactéries Gram-négatif, et en particulier les bactéries psychotropes.

10. Procédé selon l'une quelconque des revendications 1 à 9, dans lequel la bactérie receveuse est non productrice de nisine et sensible à la nisine, et dans lequel l'ADN destiné à être introduit, est étranger à la bactérie receveuse, et code pour une production de nisine, et il est intégré dans le chromosome ou à l'extérieur du chromosome dans le micro-organisme producteur de nisine résultant.

11. Procédé selon la revendication 10, dans lequel la bactérie receveuse dans laquelle l'ADN doit être transféré, est inhibée par la nisine, et dans lequel le micro-organisme modifié producteur de nisine, n'est pas inhibé par la nisine.

12. Procédé de conservation d'une matière en incorporant un micro-organisme dans la matière, caractérisé en ce qu'on incorpore, dans la matière, un micro-organisme modifié producteur de nisine produit selon l'une quelconque des revendications 1 à 11.

**13.** Procédé selon la revendication 12, dans lequel la matière est une quelconque substance sujette à une dégradation ou une altération, choisie parmi les matières agricoles, industrielles et alimentaires.

**14.** Procédé de production de nisine, dans lequel on cultive un micro-organisme modifié producteur de nisine, obtenu selon l'une quelconque des revendications 1 à 11, et on isole la nisine à partir du milieu de culture.

**15.** Procédé selon la revendication 14, dans lequel la nisine isolée, est caractérisée selon un procédé d'analyse biologique mettant en oeuvre Streptococcus cremoris ATCC 14365 sensible à la nisine.

**16.** Procédé de production de nisine contenant en outre d'autres substances inhibitrices, dans lequel on cultive un micro-organisme modifié producteur de nisine obtenu selon l'une quelconque des revendications 1 à 11, et on isole la nisine et les autres substances inhibitrices à partir du milieu de culture.

**17.** Procédé de production du micro-organisme modifié producteur de nisine, obtenu selon l'une quelconque des revendications 1 à 11, sous forme concentrée, d'une culture liquide non concentrée, d'une culture concentrée congelée ou non congelée, d'une culture séchée stabilisée et d'une culture lyophilisée, de façon connue en soi.

**18.** Procédé de production du micro-organisme modifié producteur de nisine, obtenu selon l'une quelconque des revendications 1 à 11, sous forme non concentrée, de façon connue en soi.

**19.** Procédé de production du micro-organisme modifié producteur de nisine obtenu selon l'une quelconque des revendications 1 à 11, sous forme concentrée comprenant une poudre non lyophilisée et congelée ou non congelée, une poudre lyophilisée, ou une poudre sèche stabilisée et non lyophilisée, de façon connue en soi.

**20.** Procédé de préparation d'une composition dans lequel le micro-organisme modifié producteur de nisine obtenu selon l'une quelconque des revendications 1 à 11, est mélangé avec un micro-organisme non producteur de nisine.

**21.** Procédé selon la revendication 20, dans lequel le micro-organisme modifié producteur de nisine obtenu selon l'une quelconque des revendications 1 à 11, est mélangé avec Streptococcus lactis de la sous-espèce diacetilactis non producteur de nisine, qui a été sélectionné eu égard à la résistance à la nisine.

**22.** Procédé de préparation d'une composition, dans lequel le micro-organisme modifié producteur de nisine obtenu selon l'une quelconque des revendications 1 à 11, est mélangé avec des micro-organismes modifiés producteurs de nisine, ayant différentes sensibilités à l'égard des phages, de façon à permettre la production assurée de nisine sans précautions rigoureuses contre les bactériophages.

**23.** Utilisation de micro-organismes producteurs de nisine obtenus selon l'une quelconque des revendications 1 à 11, pour la préparation d'une composition contenant les micro-organismes seuls ou en mélange avec d'autres micro-organismes, destinée à une utilisation pour le traitement d'animaux.

**24.** Procédé amélioré de conservation d'un produit alimentaire frais ou fermenté, selon lequel on mélange un micro-organisme avec l'aliment, caractérisé en ce qu'on maintient l'aliment à des températures de réfrigération, en présence du micro-organisme modifié producteur de nisine obtenu selon l'une quelconque des revendications 1 à 11.

**25.** Procédé de préparation d'une composition bactérienne, selon lequel on mélange les composants :
    (a) un Streptococcus producteur de nisine ; et
    (b) un Streptococcus diacetilactis résistant à la nisine, les bactéries dans la composition, étant sous forme biologiquement pure.

**26.** Procédé selon la revendication 25, dans lequel le Streptococcus lactis producteur de nisine, est naturel.

**27.** Procédé selon la revendication 25, dans lequel le mélange est préservé, et contient au moins environ $10^6$ cellules par ml.

**28.** Procédé selon la revendication 25, dans lequel le mélange est préservé et contient au moins environ $10^9$ cellules par ml.

**29.** Procédé d'élaboration d'un micro-organisme modifié producteur de nisine, selon lequel :
   (a) on transfère un ADN qui code pour la production de nisine à partir d'un Streptococcus donneur producteur de nisine, à une bactérie receveuse qui produit également de la nisine, par conjugaison d'une bactérie donneuse avec une bactérie receveuse, résultant en un micro-organisme modifié producteur de nisine ayant une capacité de production de nisine accrue ; et
   (b) on isole le micro-organisme modifié producteur de nisine obtenu.

**30.** Micro-organisme modifié producteur de nisine, contenant un ADN étranger chromosomique ou extra-chromosomique, qui code pour la production de nisine, cet ADN étranger étant obtenu à partir d'un Streptococcus donneur producteur de nisine, ce micro-organisme modifié pouvant être obtenu par conjugaison d'une bactérie donneuse productrice de nisine avec une bactérie receveuse sensible à la nisine, résultant en le micro-organisme modifié producteur de nisine.

**31.** Micro-organisme modifié producteur de nisine selon la revendication 30, la bactérie receveuse dans laquelle l'ADN a été transféré, étant choisie parmi les espèces des genres Streptococcus, Pediococcus, Lactobacillus, Propionibacterium, Micrococcus, Leuconostoc, Staphylococcus, Clostridium, Flavobacterium, Brevibacterium, l'espèce E. coli et les espèces du genre Pseudomonas.

**32.** Micro-organisme modifié producteur de nisine selon la revendication 30, le micro-organisme donneur à partir duquel l'ADN a été transféré, étant une souche de Streptococcus lactis productrice de nisine.

**33.** Micro-organisme modifié producteur de nisine selon la revendication 30, le micro-organisme donneur à partir duquel l'ADN a été transféré, étant Streptococcus lactis, et le micro-organisme receveur étant Streptococcus diacetilactis.

**34.** Micro-organisme modifié producteur de nisine selon la revendication 30, les micro-organismes donneurs à partir desquels l'ADN a été transféré, étant choisis parmi Streptococcus lactis ATCC 11 454, NRRL-B-15 458, NRRL-B-15 459, NRRL-B-15 460, NRRL-B-15 461, NRRL-B-15 462 et NRRL-B-15 470, et leurs dérivés isogéniques.

**35.** Micro-organisme modifié producteur de nisine selon la revendication 30, la bactérie receveuse étant choisie parmi Streptococcus lactis de la sous-espèce diacetilactis NRRL-B-15 005, NRRL-B-15 006, NRRL-B-15018, NRRL-B-12 070 et NRRL-B-12 071, et leurs dérivés isogéniques et/ou parmi Streptococcus lactis NRRL-B-11 452, NRRL-B-15 453 et NRRL-B-15 454, et leurs dérivés isogéniques.

**36.** Micro-organisme modifié producteur de nisine selon la revendication 30, dans lequel la bactérie receveuse dans laquelle l'ADN a été transféré, produisant des composés inhibiteurs de bactéries Gram-négatif, et en particulier les bactéries psychotropes, et le Streptococcus donneur à partir duquel l'ADN a été transféré, produisant de la nisine, ces deux produits étant formés par le micro-organisme producteur de nisine.

**37.** Micro-organisme modifié producteur de nisine selon la revendication 36, qui inhibe les bactéries Gram-positif et en particulier les bactéries sporogènes, et qui inhibe également les bactéries Gram-négatif, en particulier les bactéries psychotropes.

**38.** Micro-organisme modifié producteur de nisine selon la revendication 36, choisi parmi les Stroptococcus lactis de la sous-espèce diacetilactis modifiés NRRL-B-15 464, NRRL-B-15 465, NRRL-B-15 466, NRRL-B-15 467, NRRL-B-15 468 et NRRL-B-15 469, et leurs dérivés bactériens isogéniques.

**39.** Micro-organisme modifié producteur de nisine selon l'une quelconque des revendications 30 à 38, la bactérie receveuse étant non productrice de nisine et sensible à la nisine, et l'ADN introduit étant étranger à la bactérie receveuse, et codant pour une production de nisine, et il est intégré dans le

32

chromosome ou à l'extérieur du chromosone dans le micro-organisme producteur de nisine résultant.

40. Micro-organisme modifié producteur de nisine selon la revendication 39, la bactérie receveuse dans laquelle l'ADN a été transféré, étant inhibée par la nisine, et le micro-organisme modifié producteur de nisine, n'étant pas inhibé par la nisine.

41. Micro-organisme modifié producteur de nisine selon l'une quelconque des revendications 30 à 40, sous forme concentrée, d'une culture liquide non concentrée, d'une culture concentrée congelée ou non congelée, d'une culture séchée et stabilisée et d'une culture lyophilisée.

42. Micro-organisme producteur de nisine selon l'une quelconque des revendications 30 à 40, sous forme non concentrée.

43. Micro-organisme modifié producteur de nisine selon l'une quelconque des revendications 30 à 40, sous forme concentrée, comprenant une poudre non lyophilisée et congelée ou non congelée, une poudre lyophilisée ou une poudre sèche non lyophilisée et stabilisée.

44. Micro-organisme modifié producteur de nisine selon l'une quelconque des revendications 30 à 40, mélangé avec un micro-organisme non producteur de nisine.

45. Micro-organisme modifié producteur de nisine selon l'une quelconque des revendications 30 à 40, mélangé avec Streptococcus lactis de la sous-espèce diacetilactis non producteur de nisine, ayant été sélectionné eu égard à la résistance à la nisine.

46. Micro-organisme modifié producteur de nisine selon l'une quelconque des revendications 30 à 40, mélangé avec des micro-organismes modifiés producteurs de nisine, ayant différentes sensibilités aux phages, de façon à permettre la production assurée de nisine sans précautions rigoureuses contre les bactériophages.

47. Composition bactéricide, comprenant en mélange :
   (a) un Streptococcus producteur de nisine ; et
   (b) un Streptococcus diacetilactis résistant à la nisine selon la revendication 4, les bactéries incorporées dans la composition étant sous forme biologiquement pure.

48. Composition selon la revendication 47, dans laquelle le Streptococcus lactis producteur de nisine, est naturel.

49. Composition selon la revendication 47, sous forme préservée contenant au moins environ $10^6$ cellules par ml.

50. Composition selon la revendication 47, sous forme préservée contenant au moins environ $10^9$ cellules par ml.